# EUROPEAN PATENT APPLICATION

(11) **EP 2 364 704 A1**
(43) Date of publication of application: **14.09.2011**
(21) Application number: 11163020.8
(22) Date of filing: 06.02.2008
(51) Int. Cl.: A61K 31/428, A61K 45/06

(54) **Combination of beta-adrenoceptor agonist and corticosteroid**

(30) Priority: 08.02.2007 GB 0702456
(62) Divisional of application: 08702047.5
(71) Applicant: AstraZeneca AB, 151 85 Södertälje (SE)
(72) Inventor: Cadogan, Elaine, Bridget, Loughborough, Leicestershire LE11 5RH (GB); Connolly, Stephen, Loughborough, Leicestershire LE11 5RH (GB); Nicholls, David, John, Macclesfield, Cheshire SK10 4TG (GB); Wiley, Katherine, Elisabeth, Loughborough, leicestershire LE11 5RH (GB); Young, Alan, Loughborough, leicestershire LE11 5RH (GB)

(57) **Abstract**

The invention provides a pharmaceutical product comprising a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a salt thereof, and a second active ingredient which is a corticosteroid.

## Description

The present invention relates to a combination of two or more pharmaceutically active substances for use in the treatment of respiratory diseases (for example chronic obstructive pulmonary disease (COPD) or asthma).

The essential function of the lungs requires a fragile structure with enormous exposure to the environment, including pollutants, microbes, allergens, and carcinogens. Host factors, resulting from interactions of lifestyle choices and genetic composition, influence the response to this exposure. Damage or infection to the lungs can give rise to a wide range of diseases of the respiratory system (or respiratory diseases). A number of these diseases are of great public health importance. Respiratory diseases include Acute Lung Injury, Acute Respiratory Distress Syndrome (ARDS), occupational lung disease, lung cancer, tuberculosis, fibrosis, pneumoconiosis, pneumonia, emphysema, Chronic Obstructive Pulmonary Disease (COPD) and asthma.

Among the most common of the respiratory diseases is asthma. Asthma is generally defined as an inflammatory disorder of the airways with clinical symptoms arising from intermittent airflow obstruction. It is characterised clinically by paroxysms of wheezing, dyspnea and cough. It is a chronic disabling disorder that appears to be increasing in prevalence and severity. It is estimated that 15% of children and 5% of adults in the population of developed countries suffer from asthma. Therapy should therefore be aimed at controlling symptoms so that normal life is possible and at the same time provide basis for treating the underlying inflammation.

COPD is a term which refers to a large group of lung diseases which can interfere with normal breathing. Current clinical guidelines define COPD as a disease state characterized by airflow limitation that is not fully reversible. The airflow limitation is usually both progressive and associated with an abnormal inflammatory response of the lungs to noxious particles and gases. The most important contributory source of such particles and gases, at least in the western world, is tobacco smoke. COPD patients have a variety of symptoms, including cough, shortness of breath, and excessive production of sputum; such symptoms arise from dysfunction of a number of cellular compartments, including neutrophils, macrophages, and epithelial cells. The two most important conditions covered by COPD are chronic bronchitis and emphysema.

Chronic bronchitis is a long-standing inflammation of the bronchi which causes increased production of mucous and other changes. The patients' symptoms are cough and expectoration of sputum. Chronic bronchitis can lead to more frequent and severe respiratory infections, narrowing and plugging of the bronchi, difficult breathing and disability.

Emphysema is a chronic lung disease which affects the alveoli and/or the ends of the smallest bronchi. The lung loses its elasticity and therefore these areas of the lungs become enlarged. These enlarged areas trap stale air and do not effectively exchange it with fresh air. This results in difficult breathing and may result in insufficient oxygen being delivered to the blood. The predominant symptom in patients with emphysema is shortness of breath.

Therapeutic agents used in the treatment of respiratory diseases include corticosteroids. Corticosteroids (also known as glucocorticosteroids or glucocorticoids) are potent antiinflammatory agents. Whilst their exact mechanism of action is not clear, the end result of corticosteroid treatment is a decrease in the number, activity and movement of inflammatory cells into the bronchial submucosa, leading to decreased airway responsiveness. Corticosteroids may also cause reduced shedding of bronchial epithelial lining, vascular permeability, and mucus secretion. Whilst corticosteroid treatment can yield important benefits, the efficacy of these agents is often far from satisfactory, particularly in COPD. Moreover, whilst the use of steroids may lead to therapeutic effects, it is desirable to be able to use steroids in low doses to minimise the occurrence and severity of undesirable side effects that may be associated with regular administration. Recent studies have also highlighted the problem of the acquisition of steroid resistance amongst patients suffering from respiratory diseases. For example, cigarette smokers with asthma have been found to be insensitive to short term inhaled corticosteroid therapy, but the disparity of the response between smokers and non-smokers appears to be reduced with high dose inhaled corticosteroid (Tomlinson et al., Thorax 2005;60:282-287).

A further class of therapeutic agent used in the treatment of respiratory diseases are bronchodilators. Bronchodilators may be used to alleviate symptoms of respiratory diseases by relaxing the bronchial smooth muscles, reducing airway obstruction, reducing lung hyperinflation and decreasing shortness of breath. Types of bronchodilators in clinical use include β₂ adrenoceptor agonists, muscarinic receptor antagonists and methylxanthines. Bronchodilators are prescribed mainly for symptomatic relief and they are not considered to alter the natural history of respiratory diseases.

*N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide and its dihydrochloride and dihydrobromide salts are β2 adrenoceptor agonists and are disclosed in PCT/SE2006/000927 (published as WO 2007/018461, see Examples 7, 15 and 16). The compound and its salts show at least a 10-fold selectivity of β2 adrenoceptor agonism over adrenergic α1D, adrenergic β1 and dopamine D2 activities.

Combination products comprising a β₂ adrenoceptor agonist and a corticosteroid are available. One such product is a combination of budesonide and formoterol fumarate (marketed by AstraZeneca under the tradename Symbicort ®), which has proven to be effective in controlling asthma and COPD, and improving quality of life in many patients.

In view of the complexity of respiratory diseases such as asthma and COPD, it is unlikely that any one mediator can satisfactorily treat the disease alone. Moreover, whilst combination treatments using a β₂ adrenoceptor agonist and a corticosteroid deliver significant patient benefits, there remains a medical need for new therapies against respiratory diseases such as asthma and COPD, in particular for therapies with disease modifying potential.

Accordingly, the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl] amino} ethyl)-3-[2-(1-naphthyl)ethoxy]propanamide or a salt thereof, and a second active ingredient selected from:
a non-steroidal Glucocorticoid Receptor (GR Receptor) Agonist;
an antioxidant;
a CCR1 antagonist;
a chemokine antagonist (not CCR1);
a corticosteroid;
a CRTh2 antagonist;
a DP1 antagonist;
an Histone Deacetylase Inducer;
an IKK2 inhibitor;
a COX inhibitor;
a lipoxygenase inhibitor;
a leukotriene receptor antagonist;
an MPO inhibitor;
a muscarinic antagonist which is Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-(2-hydroxy-2,2-dithien-2-ylacetoxy)-1-(3-phenoxypropyl)-1-azoniabicyclo[2.2.2]octane bromide, 3(R)-1-phenethyl-3-(9H-xanthene-9-carbonyloxy)-1-azoniabicyclo[2.2.2]octane bromide or (3R)-3-[(2S)-2-cyclopentyl-2-hydroxy-2-thien-2-ylacetoxy]-1-(2-phenoxyethyl)-1-azoniabicyclo[2.2.2]actane bromide;
a p38 inhibitor;
a PDE inhibitor;
a PPARγ agonist;
a protease inhibitor;
a Statin;
a thromboxane antagonist;
a vasodilator; or,
an ENAC blocker (Epithelial Sodium-channel blocker).

In one particular aspect the present invention provides a pharmaceutical product wherein the first and second active ingredients are in forms suitable for oral administration (for example for delivery to the lungs and/or airways).

The pharmaceutical product of the present invention comprises a first active ingredient and a second active ingredient, and it may comprise a third active ingredient. The third active ingredient can be chosen from the list of second active ingredients but would normally have a different mechanism of action. So, for example, the second active ingredient might be a muscarinic antagonist and the third active ingredient might be: a non-steroidal glucocorticosteroid receptor agonist, corticosteroid, a CCR1 antagonist or a PDE4 inhibitor.

A suitable salt of *N*-[2-(Diethylamino)ethyl]-*N*-(2-{[2-(4-hydroxy-2-oxo-2,3-dihydro-1,3-benzothiazol-7-yl)ethyl] amino }ethyl)-3-[2-(1-naphthyl)ethoxy]propanamide is, for example, a hydrochloride, hydrobromide (such as dihydrobromide), trifluoroacetate, sulphate, phosphate, acetate, fumarate, maleate, tartrate, lactate, citrate, pyruvate, succinate, oxalate, methanesulphonate, p-toluenesulphonate, bisulphate, benzenesulphonate, ethanesulphonate, malonate, xinafoate, ascorbate, oleate, nicotinate, saccharinate, adipate, formate, glycolate, L-lactate, D-lactate, aspartate, malate, L-tartrate, D-tartrate, stearate, 2-furoate, 3-furoate, napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), isethionate (2-hydroxyethylsulfonate), 2-mesitylenesulphonate and 2-naphthalenesulphonate.

In one aspect the present invention provides a pharmaceutical product wherein the first active ingredient is *N*-[2-(Diethylamino)ethyl]-*N*-(2-{[2-(4-hydroxy-2-oxo-2,3-dihydro-1,3 -benzothiazol-7-yl)ethyl] amino}ethyl)-3-[2-(1-naphthyl)ethoxy]propanamide dihydrobromide.

The first and second active ingredients can be administered simultaneously (either in a single pharmaceutical preparation {that is, the active ingredients are in admixture} or via separate preparations), or sequentially or separately via separate pharmaceutical preparations.

A non-steroidal glucocorticoid receptor (GR) agonist is, for example, a compound disclosed in WO 2006/046916.

An antioxidant is, for example, Allopurinol, Erdosteine, Mannitol, N-acetyl cysteine choline ester, N-acetyl cysteine ethyl ester, N-Acetylcysteine, N-Acetylcysteine amide or Niacin.

A CCR1 antagonist is, for example, a compound disclosed in WO2001/062728 or WO2001/098273, or a pharmaceutically acceptable salt thereof (such as a hydrochloride, trifluoroacetate, sulphate, (hemi)fumarate, benzoate, furoate or succinate salt); BX471 ((2R)-1-[[2-[(aminocarbonyl)amino]-4-chlorophenoxy]acetyl]-4-[(4-fluorophenyl)methyl]-2-methylpiperazine monohydrochloride) or CCX634.

Also, a CCR1 antagonist is, for example, a compound disclosed in WO2001/062728 or WO2001/098273 [such as N-(2{(2S)-3[{(3R)-1-[(4-chlorophenyl)methyl]-3-pyrrolidinyl} amino]-2-hydroxypropoxy} -4-fluorophenyl)acetamide, N-(2{(2S)-3[{(3S)-1-[(4-chlorophenyl)methyl]-3-pyrrolidinyl}amino]-2-hydroxypropoxy}-4-fluorophenyl)acetamide, N-(2-{(2S)-3-[1-{(4-chlorobenzoyl)-4-piperidinyl}amino]-2-hydroxypropoxy}-4-hydroxyphenyl)acetamide, (2-{[(2S)-3-{[(2R,5S)-1-(4-chlorobenzyl)-2,5-dimethylpiperidin-4-yl]amino}-2-hydroxy-2-methylpropyl]oxy}-4-fluorophenyl)acetic acid, (2- {[(2S)-3-{[(3S,4R)-1-(4-chlorobenzyl)-3-methylpiperidin-4-yl]amino}-2-hydroxy-2-methylpropyl]oxy}-4-fluorophenyl)acetic acid, (2-{[(2S)-3-{[(3R,4R)-1-(4-chlorobenzyl)-3-methylpiperidin-4-yl]amino}-2-hydroxy-2-methylpropyl]oxy}-4-fluorophenyl)acetic acid, (2-{[(2S)-3-{[(2R,4S,5S)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2R,4R,SS)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2S,4R,5R)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2S,4S,5R)-1-(4-chlorobenzyl)-2,5-dimethylpiperidin-4-yl]amino}-2-hydroxy-2-methylpropyl]oxy}-4-fluorophenyl)acetic acid, Methyl (2-{[(2*S*)-3-{[1-(4-chlorobenzyl)piperidin-4-yl]amino}-2-hydroxypropyl]oxy}-4-fluorophenyl)propanoate, N-[2-({2S}-3-[(1-[4-chlorobenzyl]-4-piperidinyl)amino]-2-hydroxypropoxy)-4-chlorophenyl acetamide, N-[2-({2S}-3-[(1-[4-chlorobenzyl]-4-piperidinyl)amino]-2-hydroxy-2-methylpropoxy)-4-hydroxyphenyl] acetamide, N-[2-({2S}-3-[(1-[4-chlorobenzyl]-4-piperidinyl)amino]-2-hydroxy-2-methylpropoxy)-4-fluorophenyl] acetamide, N-[5-chloro-[2-({2S}-3-[(1-[4-chlorobenzyl]-4-piperidinyl)amino]-2-hydroxy-2-methylpropoxy)-4-hydroxyphenyl] acetamide, N-[5-chloro-[2-({2S}-3-[(1-[4-chlorobenzyl]-4-piperidinyl)amino]-2-hydroxy-2-methylpropoxy)-4-hydroxyphenyl] propaneamide, (2-{[(2*S*)-3-{[1-(4-chlorobenzyl)piperidin-4-yl]amino}-2-hydroxy-2-methylpropyl]oxy}-4-fluorophenyl)methanesulfonic acid, N-5-chloro-(2-{(2S)-3-[1-{(4-chlorobenzyl)-4-pipendinyl}amino]-2-hydroxypropoxy} -4-hydroxyphenyl)-N'-cyclopropyl-urea, N-(2-{(2S)-3-[1-{(4-chlorobenzyl)-4-pipendmyl}amino]-2-hydroxypropoxy}-phenyl)-N'-ethyl-urea, (2S)-1-(2-ethylphenoxy)-3[(1-[4-chlorobenzyl]4-piperidinyl)amino]propan-2-ol, (2S)-1-[2-(-hydroxyethyl)phenoxy]-2-methyl-3[(1-[4-chlorobenzyl]-4-piperidinyl)amino]propan-2-ol, 2-({2S}-3-[(1-[4-chlorobenzyl]-4-piperidiyl)amino]-2-hydroxy-2-methylpropoxy)benzaldehyde, 2-({2S}-3-[(1-[4-chlorobenzyl]-4-piperidinyl)amino]-2-hydroxypropoxy)-N-cyclopropylbenzamide, Methyl 2-({2S}-3-[(1-[4-chlorobenzyl]-4-piperidinyl)amino]-2-hydroxypropoxy)-4-fluorobenzoate, N-(2-{[(2S)-3-(5-chloro-1'H,3H-spiro[1-benzofuran-2,4'-piperidin]-1'-yl)-2-hydroxypropyl]oxy}-4-hydroxyphenyl)acetamide, N-(2-{[(2S)-3-(5-chloro-1'H-spiro[1,3-benzodioxole-2,4'-piperidin]-1'-yl)-2-hydroxypropyl]oxy}-4-hydroxyphenyl)acetamide, 2-{[(2S)-3-(5-chloro-1'H,3H-spiro[1-benzofuran-2,4'-piperidin]-1'-yl)-2-hydroxypropyl]oxy}-4-hydroxy-N-methylbenzamide, 2-{[(2S)-3-(5-chloro-1'H,3H-spiro[1-benzofuran-2,4'-piperidin]-1'-yl)-2-hydroxypropyl]oxy}-4-hydroxybenzoic acid, N-(2-{[(2S)-3-(5-chloro-1'H,3H-spiro[2-benzofuran-1,4'-piperidin]-1'-yl)-2-hydroxypropyl]oxy}-4-hydroxyphenyl)acetamide; 2-{[(2S)-3-(5-chloro-1'H,3H-spiro[2-benzofuran-1,4'-piperidin]-1'-yl)-2-hydroxypropyl]oxy}-4-hydroxy-N-methylbenzamide, N-(2-{[(2S)-3-(5-fluoro-1'H,3H-spiro[1-benzofuran-2,4'-piperidin]-1'-yl)-2-hydroxypropyl]oxy}-4-hydroxyphenyl)acetamide, 2-{[(2S)-3-(5-fluoro-1'H,3H-spiro[1-benzofuran-2,4'-piperidin]-1'-yl)-2-hydroxypropyl]oxy}-4-hydroxy-N-methylbenzamide, N-[2-({(2S)-3-[(2R)-5-chloro-1'H,3H-spiro 1-benzofuran-2,3'-pyrrolidin]-1'-yl]-2-hydroxypropyl}oxy)-4-hydroxyphenyl]acetamide, N-(2-{[(2S)-3-(5-chloro-1'H,3H-spiro[1-benzofuran-2,4'-piperidin]-1'-yl)-2-hydroxypropyl]oxy}-4-hydroxyphenyl)urea, 4-fluoro-2-{[(2S)-3-(5-fluoro-1'H,3H-spiro[1-benzofuran-2,4'-pipendin]-1'-yl)-2-hydroxypropyl]oxy}benzoic acid, N-(2-{[(2S)-3-(5-chloro-1'H,3H-spiro[1-benzofuran-2,4'-piperidin]-1'-yl)-2-hydroxypropyl]oxy}-4-fluorophenyl)urea, N-(2-{[(2S)-2-amino-3-(5-fluoro-1'H,3H-spiro[1-benzofuran-2,4'-piperidin]-1'-yl)propyl]oxy}-4-hydroxyphenyl)acetamide, 2-[(2S)-3-(5-chlorospiro[benzofuran-2(3H),4'-piperidin]-1'-yl)-2-hydroxypropoxy]-benzaldehyde, (αS)-5-chloro-a-[[2-(2-hydroxyethyl)phenoxy]methyl]-Spiro[benzofuran-2(3H),4'-piperidine]-1'-ethanol, (αS)-5-chloro-a-[[2-(hydroxymethyl)phenoxy]methyl]-Spiro[benzofuran-2(3H),4'-piperidine]-1'-ethanol, N-(2- {[(2S)-3-(5-chloro-1'H,3H-spiro[1-benzofuran-2,4'-piperidin]-1'-yl)-2-hydroxypropyl]oxy}-5-chloro-4-hydroxyphenyl)acetamide, 2-Chloro-5- {[(2*S*)-3-(5-chloro-1'*H*,3*H*-spiro[1'-benzofuran-2,4'-piperidin]-1'-yl)-2-hydroxypropyl]oxy}-(4-{acetylammo}phenoxy)acetic acid, 5-{[(2*S*)-3-(5-Chloro-1'*H*,3*H*-spiro[1-benzofuran-2,4'-piperidin]-1'-yl)-2-hydroxypropyl]oxy}-(4-{acetylamino}phenoxy)acetic acid, {2-Chloro-5-{[(2*S*)-3-(5-chloro-1'*H*,3*H*-spiro[1-benzofuran-2,4'-piperidin]-1'-yl)-2-hydroxypropyl]oxy}-4-[(methylamino)carbonyl]phenoxy}acetic acid, 2- {2-Chloro-5-{[(2*S*)-3-(5-chloro-1'*H*,3*H-*spiro[1-benzofuran-2,4'-piperidin]-1'-yl)-2-hydroxypropyl]oxy}-4-[(methylamino)carbonyl]phenoxy}-2-methylpropanoic acid, (2-Chloro-5-{[(2*S*)-3-(5-chloro-1'*H*,3*H*-spiro[1-benzofuran-2,4'-piperidin]-1'-yl)-2-hydroxypropyl]oxy}-4-{[(36)-3-hydroxypyrrolidin-1-yl]carbonyl}phenoxy)acetic acid, 5-Chloro-2-{[(2*S*)-3-(5-chloro-1'*H*,3*H*-spiro[1-benzofuran-2,4'-piperidin]-1'-yl)-2-hydroxypropyl]oxy}-4-(cyanomethoxy)benzoic acid, 2-{[(2S)-3-(5-chloro-1'H,3H-spiro[1-benzofnran-2,4'-piperidin]-1'-yl)-2-hydroxypropyl]oxy}-5-chloro-4-(2,2-difluoroethoxy)benzoic, 5-Chloro-2- {[(2*S*)-3-(5-chloro-1'*H*,3*H*-spiro[1-benzofuran-2,4'-piperidin]-1'-yl)-2-hydroxypropyl]oxy}-4-(3,3,3-trifluoropropoxy)benzoic acid, N-(2-{3-[5-chloro-1'H,3H-spiro[1-benzofuran-2,4'-piperidin]-1'-yl]propoxy}phenyl)acetamide, Methyl 3-(2-{[(2S)-3-(5-chloro-1'*H*3*H*-spiro[1-benzofuran-2,4'-piperidin]-1'-yl)-2-hydroxypropyl]oxy}-4-fluorophenyl)propanoic acid, N-(2-{[(2S)-3-({spiro[indole-2-4'-piperidin]-3(1H)-one}-1'-yl)-2-hydroxypropyl]oxy}-4-hydroxyphenyl)acetamide, or (2-{[(2*S*)-3-(5-Chloro-1'*H*,3*H-*spiro[1-benzofuran-2,4'-piperidin]-1'-yl)-2-hydroxypropyl]oxy}-4-fluorophenyl)methanesulfonic acid, or a pharmaceutically acceptable salt thereof (for example as described above; (such as a hydrochloride, trifluoroacetate, sulphate, (hemi)fumarate, benzoate, furoate or succinate salt))]; BX471 ((2R)-1-[[2-[(aminocarbonyl)amino]-4-chlorophenoxy]acetyl]-4-[(4-fluorophenyl)methyl]-2-methylpiperazine monohydrochloride); or CCX634.

Also, a CCR1 antagonist is, for example, *N*-{2-[((2*S*)-3-{[1-(4-chlorobenzyl)piperidin-4-yl]amino} -2-hydroxy-2-methylpropyl)oxy]-4-hydroxyphenyl} acetamide (see WO 2003/051839), or, 2-{2-Chloro-5-{[(2S)-3-(5-chloro-1'H,3H-spiro[1-benzofuran-2,4'-piperidin]-1'-yl)-2-hydroxypropyl]oxy}-4-[(methylamino)carbonyl]phenoxy}-2-methylpropanoic acid (see PCT publication no. WO 2008/010765), or a pharmaceutically acceptable salt thereof (for example a hydrochloride, sulphate, (hemi)fumarate, benzoate, furoate or succinate salt).

A chemokine antagonist (other than a CCR1 antagonist), for example, 656933 (N-(2-bromophenyl)-N'-(4-cyano-1H-1,2,3-benzotriazol-7-yl)urea), 766994 (4-({[({[(2R)-4-(3,4-dichlorobenzyl)morpholin-2-yl]methyl} amino)carbonyl]-amino}methyl)benzamide), CCX-282, CCX-915, Cyanovirin N, E-921, INCB-003284, INCB-9471, Maraviroc, MLN-3701, MLN-3897, T-487 (N-{1-[3-(4-ethoxyphenyl)-4-oxo-3,4-dihydropyrido[2,3-d]pyrimidin-2-yl] ethyl}-N-(pyridin-3-ylmethyl)-2-[4-(trifluoromethoxy)phenyl]acetamide) or Vicriviroc.

A corticosteroid is, for example, Alclometasone dipropionate, Amelometasone, Beclomethasone dipropionate, Budesonide, Butixocort propionate, Ciclesonide, Clobetasol propionate, Desisobutyrylciclesonide, Etiprednol dicloacetate, Fluocinolone acetonide, Fluticasone Furoate, Fluticasone propionate, Loteprednol etabonate (topical) or Mometasone furoate.

A CRTh2 antagonist is, for example, a compound from WO 2004/106302 or WO 2005/018529.

A DP1 antagonist is, for example, L888839 or MK0525.

An histone deacetylase inducer is, for example, ADC4022, Aminophylline, a Methylxanthine or Theophylline.

An IKK2 inhibitor is, for example, 2-{[2-(2-Methylamino-pyrimidin-4-yl)-1H-indole-5-carbonyl]-amino}-3-(phenyl-pyridin-2-yl-amino)-propionic acid.

A COX inhibitor is, for example, Celecoxib, Diclofenac sodium, Etodolac, Ibuprofen, Indomethacin, Meloxicam, Nimesulide, OC1768, OC2125, OC2184, OC499, OCD9101, Parecoxib sodium, Piceatannol, Piroxicam, Rofecoxib or Valdecoxib.

A lipoxygenase inhibitor is, for example, Ajulemic acid, Darbufelone, Darbufelone mesilate, Dexibuprofen lysine (monohydrate), Etalocib sodium, Licofelone, Linazolast, Lonapalene, Masoprocol, MN-001, Tepoxalin, UCB-35440, Veliflapon, ZD-2138, ZD-4007 or Zileuton ((±)-1-(1-Benzo[b]thien-2-ylethyl)-1-hydroxyurea)

A leukotriene receptor antagonist is, for example, Ablukast, Iralukast (CGP 45715A), Montelukast, Montelukast sodium, Ontazolast, Pranlukast, Pranlukast hydrate (mono Na salt), Verlukast (MK-679) or Zafirlukast.

A muscarinic antagonist is Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-(2-hydroxy-2,2-dithien-2-ylacetoxy)-1-(3-phenoxypropyl)-1-azoniabicyclo[2.2.2]octane bromide (see WO 01/04118), 3(R)-1-phenethyl-3-(9H-xanthene-9-carbonyloxy)-1-azoniabicyclo[2.2.2]octane bromide or (3R)-3-[(2S)-2-cyclopentyl-2-hydroxy-2-thien-2-ylacetoxy]-1-(2-phenoxyethyl)-1-azoniabicyclo[2.2.2]actane bromide (see WO 01/04118). In one aspect of the invention a muscarinic antagonist is Aclidinium bromide, Glycopyrrolate (such as R,R-, R,S-, S,R-, or S,S-glycopyrronium bromide), Oxitropium bromide, Pirenzepine, telenzepine or Tiotropium bromide.

An MPO Inhibitor is, for example, a Hydroxamic acid derivative (N-(4-chloro-2-methylphenyl)-4-phenyl-4-[[(4-propan-2-ylphenyl)sulfonylamino]methyl]piperidine-1-carboxamide), Piceatannol or Resveratrol.

A p38 Inhibitor is, for example, a compound from WO 2005/042502, 681323, 856553, AMG548 (2-[[(2S)-2-amino-3-phenylpropyl]amino]-3-methyl-5-(2-naphthalenyl)-6-(4-pyridinyl)-4(3H)-pyrimidinone), Array-797, Doramapimod, KC-706, PH 797804, R1503, SC-80036, SCIO469, 6-chloro-5-[[(2*S*,5*R*)-4-[(4-fluorophenyl)methyl]-2,5-domethyl-1-piperazinyl]carbonyl]-*N*,*N*,1-trimethyl-α-oxo-1*H*-indole-3-acetamide, VX702 or VX745 (5-(2,6-dichlorophenyl)-2-(phenylthio)-6H-pyrimido[1,6-b]pyridazin-6-one).

A PDE Inhibitor: such as a PDE4 inhibitor, for example, 256066, Arofylline (3-(4-chlorophenyl)-3,7-dihydro-1-propyl-1H-Purine-2,6-dione), AWD 12-281 (N-(3,5-dichloro-4-pyridinyl)-1-[(4-fluorophenyl)methyl]-5-hydroxy-a-oxo-1H-indole-3-acetamide), BAY19-8004 (Bayer), CDC-801 (Calgene), Celgene compound ((βR)-β-(3,4-dimethoxyphenyl)-1,3-dihydro-1-oxo-2H-isoindole-2-propanamide), Cilomilast (cis-4-cyano-4-[3-(cyclopentyloxy)-4-methoxyphenyl]-cyclohexanecarboxylic acid), a compound in WO2006098353 from Kyowa Hakko Kogyo Co. Ltd. Japan, 2-(3,5-dichloro-4-pyridinyl)-1-(7-methoxyspiro[1,3-benzodioxole-2,1'-cyclopentan]-4-yl)ethanone (CAS number 185406-34-2)), Compound from Pfizer (2-(3,4-difluorophenoxy)-5-fluoro-N-[cis-4-[(2-hydroxy-5-methylbenzoyl)amino]cyclohexyl]-)-3-pyridinecarboxamide), Compound from Pfizer (2-(3,4-difluorophenoxy)-5-fluoro-N-[cis-4-[[2-hydroxy-5-(hydroxymethyl)benzoyl]amino]cyclohexyl]-3-pyridinecarboxamide,), CT2820, GPD-1116, Ibudilast, IC 485, KF 31334, KW-4490 (Kyowa Hakko Kogyo), Lirimilast ([2-(2,4-dichlorobenzoyl)-6-[(methylsulfonyl)oxy]-3-benzofuranyl])-urea), Merck Compound (N-cyclopropyl-1,4-dihydro-4-oxo-1-[3-(3-pyridinylethynyl)phenyl]-)-1,8-naphthyridine-3-carboxamide), Oglemilast (N-(3,5-dichloro-4-pyridinyl)-4-(difluoromethoxy)-8-[(methylsulfonyl)amino])-1-dibenzofurancarboxamide), ON06126, ORG 20241 (4-(3,4-dimethoxyphenyl)-N-hydroxy-)-2-thiazolecarboximidamide), PD189659/PD168787 (Parke-Davis), Pentoxifylline (3,7-dihydro-3,7-dimethyl-1-(5-oxohexyl)-)-1H-purine-2,6-dione), Pfizer compound (5-fluoro-N-[4-[(2-hydroxy-4-methylbenzoyl)amino]cyclohexyl]-2-(thian-4-yloxy)pyridine-3-carboxamide), Pfizer UK 500,001, Piclamilast (3-(cyclopentyloxy)-N-(3,5-dichloro-4-pyridinyl)-4-methoxybenzamide), PLX-369 (WO 2006026754), Roflumilast (3-(cyclopropylmethoxy)-N-(3,5-dichloro-4-pyridinyl)-4-(difluoromethoxy)benzamide), SCH 351591 (N-(3,5-dichloro-1-oxido-4-pyridinyl)-8-methoxy-2-(trifluoromethyl)-5-quinolinecarboxamide), SelCID(TM) CC-10004 (Calgene), T-440 (Tanabe), Tetomilast (6-[2-(3,4-diethoxyphenyl)-4-thiazolyl]-2-pyridinecarboxylic acid), Tofimilast (9-cyclopentyl-7-ethyl-6,9-dihydro-3-(2-thienyl)-5H-pyrazolo[3,4-c]-1,2,4-triazolo[4,3-a]pyridine), TPI 1100, UCB 101333-3 (N,2-dicyclopropyl-6-(hexahydro-1H-azepin-1-yl)-5-methyl-4-pyrimidinamine), V-11294A

(Napp), VM554/VM565 (Vernalis), or Zardaverine (6-[4-(difluoromethoxy)-3-methoxyphenyl]-3(2H)-pyridazinone); or a PDE5 Inhibitor, for example, Gamma-glutamyl[s-(2-iodobenzyl)cysteinyl]glycine, Tadalafil, Vardenafil, sildenafil, 4-phenyl-methylamino-6-chloro-2-(1-imidazolyl)-quinazoline, 4-phenyl-methylamino-6-chloro-2-(3-pyridyl)-quinazoline, 1,3-dimethyl-6-(2-propoxy-5-methanesulphonylamidophenyl)-1,5-dihydropyrazolo[3,4-d]pyrimidin-4-one or 1-cyclopentyl-3-ethyl-6-(3-ethoxy-4-pyridyl)-pyrazolo[3,4-d]pyrimidin-4-one.

A PPARγ agonist is, for example, Pioglitazone, Pioglitazone hydrochloride, Rosiglitazone Maleate, Rosiglitazone Maleate ((-)-enantiomer, free base), Rosiglitazone maleate/Metformin hydrochloride or Tesaglitizar.

A Protease Inhibitor is, for example, Alpha1-antitrypsin proteinase Inhibitor, EPI-HNE4, UT-77, ZD-0892 or a compound from WO 2006/004532, WO 2005/026123, WO 2002/0744767 or WO 22002/074751; or a TACE Inhibitor (for example DPC-333, Sch-709156 or Doxycycline).

A Statin is, for example, Atorvastatin, Lovastatin, Pravastatin, Rosuvastatin or Simvastatin.

A Thromboxane Antagonist is, for example, Ramatroban or Seratrodast.

A Vasodilator is, for example, A-306552, Ambrisentan, Avosentan, BMS-248360, BMS-346567, BMS-465149, BMS-509701, Bosentan, BSF-302146 (Ambrisentan), Calcitonin Gene-related Peptide, Daglutril, Darusentan, Fandosentan potassium, Fasudil, Iloprost, KC-12615 (Daglutril), KC-12792 2AB (Daglutril), Liposomal treprostinil, PS-433540, Sitaxsentan sodium, Sodium Ferulate, TBC-11241 (Sitaxsentan), TBC-3214 (N-(2-acetyl-4,6-dimethylphenyl)-3-[[(4-chloro-3-methyl-5-isoxazolyl)amino]sulfonyl]-2-thiophenecarboxamide), TBC-3711, Trapidil, Treprostinil diethanolamine or Treprostinil sodium.

An ENAC (Epithelial Sodium-channel blocker) is, for example, Amiloride, Benzamil, Triamterene, 552-02, PSA14984, PSA25569, PSA23682 or AER002.

All the above active ingredients may be in the form of a solvate, for example, a hydrate.

In one particular aspect the present invention provides a pharmaceutical product comprising the first and second active ingredients in admixture. Alternatively, the pharmaceutical product may, for example, be a kit comprising a preparation of the first active ingredient and a preparation of the second active ingredient and, optionally, instructions for the simultaneous, sequential or separate administration of the preparations to a patient in need thereof.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino} ethyl)-3-[2-(1-naphthyl)ethoxy]propanamide dihydrobromide, and a second active ingredient selected from:
a non-steroidal Glucocorticoid Receptor (GR Receptor) Agonist;
a CCR1 antagonist;
a chemokine antagonist (not CCR1);
a corticosteroid;
an IKK2 inhibitor;
a muscarinic antagonist which is Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-(2-hydroxy-2,2-dithien-2-ylacetoxy)-1-(3-phenoxypropyl)-1-azoniabicyclo[2.2.2]octane bromide, 3(R)-1-phenethyl-3-(9H-xanthene-9-carbonyloxy)-1-azoniabicyclo[2.2.2]octane bromide or (3R)-3-[(2S)-2-cyclopentyl-2-hydroxy-2-thien-2-ylacetoxy]-1-(2-phenoxyethyl)-1-azoniabicyclo[2.2.2]actane bromide;
a p38 inhibitor; or,
a PDE inhibitor.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl] amino} ethyl)-3-[2-(1-naphthyl)ethoxy]propanamide dihydrobromide, and a second active ingredient which is a non-steroidal Glucocorticoid Receptor (GR) Agonist for example, a compound disclosed in WO 2006/046916.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl] amino} ethyl)-3-[2-(1-naphthyl)ethoxy]propanamide dihydrobromide, and a second active ingredient which is a CCR1 antagonist, for example, a compound disclosed in WO2001/062728 or WO2001/098273, or a pharmaceutically acceptable salt thereof (such as a hydrochloride, trifluoroacetate, sulphate, (hemi)fumarate, benzoate, furoate or succinate salt); BX471 ((2R)-1-[[2-[(aminocarbonyl)amino]-4-chlorophenoxy]acetyl]-4-[(4-fluorophenyl)methyl]-2-methylpiperazine monohydrochloride) or CCX634.

In yet another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl] amino} ethyl)-3-[2-(1-naphthyl)ethoxy]propanamide dihydrobromide, and a second active ingredient which is a CCR1 antagonist, for example, a compound disclosed in WO2001/062728 or WO2001/098273 [such as N-(2{(2S)-3[{(3R)-1-[(4-chlorophenyl)methyl]-3-pyrrolidinyl} amino]-2-hydroxypropoxy} -4-fluorophenyl)acetamide, N-(2{(2S)-3[{(3S)-1-[(4-chlorophenyl)methyl]-3-pyrrolidinyl}amino]-2-hydroxypropoxy}-4-fluorophenyl)acetamide, N-(2-{(2S)-3-[1-{(4-chlorobenzoyl)-4-piperidinyl}amino]-2-hydroxypropoxy}-4-hydroxyphenyl)acetamide, (2-{[(2S)-3-{[(2R,5S)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(3S,4R)-1-(4-chlorobenzyl)-3-methylpiperidin-4-yl]amino}-2-hydroxy-2-methylpropyl]oxy}-4-fluorophenyl)acetic acid, (2-{[(2S)-3-{[(3R,4R)-1-(4-chlorobenzyl)-3-methylpiperidin-4-yl]amino}-2-hydroxy-2-methylpropyl]oxy}-4-fluorophenyl)acetic acid, (2-{[(2S)-3-{[(2R,4S,5S)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2R,4R,5S)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2S,4R,5R)-1-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2S,4S,5R)-1-(4-chlorobenzyl)-2,5-dimethylpiperidin-4-yl]amino}-2-hydroxy-2-methylpropyl]oxy}-4-fluorophenyl)acetic acid, Methyl (2-{[(2*S*)-3-{[1-(4-chlorobenzyl)piperidin-4-yl]amino}-2-hydroxypropyl]oxy}-4-fluorophenyl)propanoate, N-[2-({2S}-3-[(1-[4-chlorobenzyl]-4-piperidinyl)amino]-2-hydroxypropoxy)-4-chlorophenyl acetamide, N-[2-({2S}-3-[(1-[4-chlorobenzyl]-4-piperidinyl)amino]-2-hydroxy-2-methylpropoxy)-4-hydroxyphenyl] acetamide, N-[2-({2S}-3-[(1-[4-chlorobenzyl]-4-piperidinyl)amino]-2-hydroxy-2-methylpropoxy)-4-fluorophenyl] acetamide, N-[5-chloro-[2-({2S}-3-[(1-[4-chlorobenzyl]-4-piperidinyl)amino]-2-hydroxy-2-methylpropoxy)-4-hydroxyphenyl] acetamide, N-[5-chloro-[2-({2S}-3-[(1-[4-chlorobenzyl]-4-piperidinyl)amino]-2-hydroxy-2-methylpropoxy)-4-hydroxyphenyl] propaneamide, (2-{[(2*S*)-3-{[1-(4-chlorobenzyl)piperidin-4-yl]amino}-2-hydroxy-2-methylpropyl]oxy}-4-fluorophenyl)methanesulfonic acid, N-5-chloro-(2-{(2S)-3-[1-{(4-chlorobenzyl)-4-pipendinyl} amino]-2-hydroxypropoxy} -4-hydroxyphenyl)-N'-cyclopropyl-urea, N-(2-{(2S)-3-[1-{(4-chlorobenzyl)-4-piperidinyl}amino]-2-hydroxypropoxy}-phenyl)-N'-ethyl-urea, (2S)-1-(2-ethylphenoxy)-3[(1-[4-chlorobenzyl]4-piperidinyl)amino]propan-2-ol, (2S)-1-[2-(-hydroxyethyl)phenoxy]-2-methyl-3[(1-[4-chlorobenzyl]-4-piperidinyl)amino]propan-2-ol, 2-({2S}-3-[(1-[4-chlorobenzyl]-4-piperidinyl)amino]-2-hydroxy-2-methylpropoxy)benzaldehyde, 2-({2S}-3-[(1-[4-chlorobenzyl]-4-piperidinyl)amino]-2-hydroxypropoxy)-N-cyclopropylbenzamide, Methyl 2-({2S}-3-[(1-[4-chlorobenzyl]-4-piperidinyl)amino]-2-hydroxypropoxy)-4-fluorobenzoate, N-(2-{[(2S)-3-(5-chloro-1'H,3H-spiro[1-benzofuran-2,4'-piperidin]-1'-yl)-2-hydroxypropyl]oxy}-4-hydroxyphenyl)acetamide, N-(2-{[(2S)-3-(5-chloro-1'H-spiro[1,3-benzodioxole-2,4'-piperidin]-1'-yl)-2-hydroxypropyl]oxy}-4-hydroxyphenyl)acetamide, 2-{[(2S)-3-(5-chloro-1'H,3H-spiro[1-benzofuran-2,4'-piperidin]-1'-yl)-2-hydroxypropyl]oxy} -4-hydroxy-N-methylbenzamide, 2-{[(2S)-3-(5-chloro-1'H,3H-spiro[1-benzofuran-2,4'-piperidin]-1'-yl)-2-hydroxypropyl]oxy}-4-hydroxybenzoic acid, N-(2-{[(2S)-3-(5-chloro-1'H,3H-spiro[2-benzofuran-1,4'-piperidin]-1'-yl)-2-hydroxypropyl]oxy}-4-hydroxyphenyl)acetamide; 2-{[(2S)-3-(5-chloro-1'H,3H-spiro[2-benzofuran-1,4'-piperidin]-1'-yl)-2-hydroxypropyl]oxy}-4-hydroxy-N-methylbenzamide, N-(2-{[(2S)-3-(5-fluoro-1'H,3H-spiro[1-benzofuran-2,4'-piperidin]-1'-yl)-2-hydroxypropyl]oxy} -4-hydroxyphenyl)acetamide, 2-{[(2S)-3-(5-fluoro-1'H,3H-spiro[1-benzofuran-2,4'-piperidin]-1'-yl)-2-hydroxypropyl]oxy}-4-hydroxy-N-methylbenzamide, N-[2-({(2S)-3-[(2R)-5-chloro-1'H,3H-spiro [1-benzofuran-2,3'-pyrrolidin]-1'-yl]-2-hydroxypropyl}oxy)-4-hydroxyphenyl]acetamide, N-(2-{[(2S)-3-(5-chloro-1'H,3H-spiro[1-benzofuran-2,4'-piperidin]-1'-yl)-2-hydroxypropyl]oxy}-4-hydroxyphenyl)urea, 4-fluoro-2-{[(2S)-3-(5-fluoro-1'H,3H-spiro[1-benzofuran-2,4'-piperidin]-1'-yl)-2-hydroxypropyl]oxy}benzoic acid, N-(2-{[(2S)-3-(5-chloro-1H,3H-spiro[1-benzofuran-2,4'-piperidin]-1'-yl)-2-hydroxypropyl]oxy}-4-fluorophenyl)urea, N-(2-{[(2S)-2-amino-3-(5-fluoro-1'H,3H-spiro[1-benzofuran-2,4'-piperidin]-1'-yl)propyl]oxy}-4-hydroxyphenyl)acetamide, 2-[(2S)-3-(5-chlorospiro[benzofuran-2(3H),4'-piperidin]-1'-yl)-2-hydroxypropoxy]-benzaldehyde, (α)-5-chloro-a-[[2-(2-hydroxyethyl)phenoxy]methyl]-Spiro[benzofuran-2(3H),4'-piperidine]-1'-ethanol, (αS)-5-chloro-a-[[2-(hydroxymethyl)phenoxy]methyl]-Spiro[benzofuran-2(3H),4'-piperidine]-1'-ethanol, N-(2-{[(2S)-3-(5-chloro-1'H,3H-spiro[1-benzofuran-2,4'-piperidin]-1'-yl)-2-hydroxypropyl]oxy}-5-chloro-4-hydroxyphenyl)acetamide, 2-Chloro-5-{[(2*S*)-3-(5-chloro-1'*H*,3*H*-spiro[1-benzofuran-2,4'-piperidin]-1'-yl)-2-hydroxypropyl]oxy}-(4-{acetylamino}phenoxy)acetic acid, 5-{[(2*S*)-3-(5-Chloro-1'*H*,3*H*-spiro[1-benzofuran-2,4'-piperidin]-1'-yl)-2-hydroxypropyl]oxy}-(4-{acetylamino}phenoxy)acetic acid, {2-Chloro-5-{[(2*S*)-3-(5-chloro-1'*H*,3*H*-spiro[1-benzofuran-2,4'-piperidin]-1'-yl)-2-hydroxypropyl]oxy}-4-[(methylamino)carbonyl]phenoxy}acetic acid, 2-{2-Chloro-5-{[(2*S*)-3-(5-chloro-1'*H*,3*H-*spiro[1-benzofuran-2,4'-piperidin]-1'-yl)-2-hydroxypropyl]oxy} -4-[(methylamino)carbonyl]phenoxy}-2-methylpropanoic acid, (2-Chloro-5-{[(2*S*)-3-(5-chloro-1'*H*,3*H*-spiro[1-benzofuran-2,4'-piperidin]-1'-yl)-2-hydroxypropyl]oxy}-4- {[(36)-3-hydroxypyrrolidin-1-yl]carbonyl}phenoxy)acetic acid, 5-Chloro-2-{[(2*S*)-3-(5-chloro-1'*H*,3*H*-spiro[1-benzofuran-2,4'-piperidin]-1'-yl)-2-hydroxypropyl]oxy}-4-(cyanomethoxy)benzoic acid, 2-{[(2S)-3-(5-chloro-1'H,3H-spiro[1-benzofuran-2,4'-piperidin]-1'-yl)-2-hydroxypropyl]oxy}-5-chloro-4-(2,2-difluoroethoxy)benzoic, 5-Chloro-2-{[(2*S*)-3-(5-chloro-1'*H*,3*H*-spiro[1-benzofuran-2,4'-piperidin]-1'-yl)-2-hydroxypropyl]oxy}-4-(3,3,3-trifluoropropoxy)benzoic acid, N-(2-{3-[5-chloro-1'H,3H-spiro[1-benzofuran-2,4'-piperidin]-1'-yl]propoxy}phenyl)acetamide, Methyl 3-(2-{[(2S)-3-(5-chloro-1'*H*3*H*-spiro[1-benzofuran-2,4'-piperidin]-1'-yl)-2-hydroxypropyl]oxy}-4-fluorophenyl)propanoic acid, N-(2-{[(2S)-3-({spiro[indole-2-4'-piperidin]-3(1H)-one}-1'-yl)-2-hydroxypropyl]oxy}-4-hydroxyphenyl)acetamide, or (2-{[(2*S*)-3-(5-Chloro-1'*H*,3*H-*spiro[1-benzofuran-2,4'-piperidin]-1'-yl)-2-hydroxypropyl]oxy} -4-fluorophenyl)methanesulfonic acid, or a pharmaceutically acceptable salt thereof (for example as described above; (such as a hydrochloride, trifluoroacetate, sulphate, (hemi)fumarate, benzoate, furoate or succinate salt))]; BX471 ((2R)-1-[[2-[(aminocarbonyl)amino]-4-chlorophenoxy]acetyl]-4-[(4-fluorophenyl)methyl]-2-methylpiperazine monohydrochloride); or CCX634.

In another aspect a CCR1 antagonist is *N*-{2-[((2*S*)-3-{[1-(4-chlorobenzyl)piperidin-4-yl]amino}-2-hydroxy-2-methylpropyl)oxy]-4-hydroxyphenyl}acetamide, or, 2-{2-Chloro-5-{[(2S)-3-(5-chloro-1'H,3H-spiro[1-benzofuran-2,4'-piperidin]-1'-yl)-2-hydroxypropyl]oxy}-4-[(methylamino)carbonyl]phenoxy}-2-methylpropanoic acid, or a pharmaceutically acceptable salt thereof (for example a hydrochloride, sulphate, (hemi)fumarate, benzoate, furoate or succinate salt). For example *N*-{2-[((2*S*)-3-{[1-(4-chlorobenzyl)piperidin-4-yl]amino}-2-hydroxy-2-methylpropyl)oxy]-4-hydroxyphenyl}acetamide as a benzoate salt, or, 2-{2-Chloro-5-{[(2S)-3-(5-chloro-1'H,3H-spiro[1-benzofuran-2,4'-piperidin]-1'-yl)-2-hydroxypropyl]oxy}-4-[(methylamino)carbonyl]phenoxy}-2-methylpropanoic acid as the free acid.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient which is a chemokine antagonist (not CCR1), for example, 656933 (N-(2-bromophenyl)-N'-(4-cyano-1H-1,2,3-benzotriazol-7-yl)urea), 766994 (4-({[({[(2R)-4-(3,4-dichlorobenzyl)morpholin-2-yl]methyl} amino)carbonyl]-amino}methyl)benzamide), CCX-282, CCX-915, Cyanovirin N, E-921, INCB-003284, INCB-9471, Maraviroc, MLN-3701, MLN-3897, T-487 (N-{1-[3-(4-ethoxyphenyl)-4-oxo-3,4-dihydropyrido[2,3-d]pyrimidin-2-yl]ethyl}-N-(pyridin-3-ylmethyl)-2-[4-(trifluoromethoxy)phenyl]acetamide) or Vicriviroc.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a corticosteroid, for example, Alclometasone dipropionate, Amelometasone, Beclomethasone dipropionate, Budesonide, Butixocort propionate, Ciclesonide, Clobetasol propionate, Desisobutyrylciclesonide, Etiprednol dicloacetate, Fluocinolone acetonide, Fluticasone Furoate, Fluticasone propionate, Loteprednol etabonate (topical) or Mometasone furoate.

In one embodiment of the present invention the corticosteroid is selected from budesonide, fluticasone propionate, fluticasone fruoate mometasone furoate, beclomethasone dipropionate or butixocort propionate ester.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a corticosteroid, for example, Budesonide, Fluticasone Furoate or Fluticasone propionate.

In one embodiment of the present invention the corticosteroid is budesonide. Budesonide and its preparation is described, for example, in Arzneimittel-Forschung (19 79), 29 (11), 1687-1690*,* DE 2,323,215 and US 3,929,768. Presently available formulations of budesonide are marketed under the tradename 'Entocort ®'.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is an IKK2 inhibitor, for example, 2-{[2-(2-Methylamino-pyrimidin-4-yl)-1H-indole-5-carbonyl]-amino}-3-(phenyl-pyridin-2-yl-amino)-propionic acid.

In yet another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a muscarinic antagonist is Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-(2-hydroxy-2,2-dithien-2-ylacetoxy)-1-(3-phenoxypropyl)-1-azoniabicyclo[2.2.2]octane bromide (see WO 01/04118), or 3(R)-1-phenethyl-3-(9H-xanthene-9-carbonyloxy)-1-azoniabicyclo[2.2.2]octane bromide or (3R)-3-[(2S)-2-cyclopentyl-2-hydroxy-2-thien-2-ylacetoxy]-1-(2-phenoxyethyl)-1-azoniabicyclo[2.2.2]actane bromide (see WO 01/04118). In one aspect of the invention a muscarinic antagonist is Aclidinium bromide, Glycopyrrolate (such as R,R-, R,S-, S,R-, or S,S-glycopyrronium bromide), Oxitropium bromide, Pirenzepine, telenzepine or Tiotropium bromide.

In yet another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a muscarinic antagonist, for example, Aclidinium bromide, Glycopyrrolate (such as R,R-, R,S-, S,R-, or S,S-glycopyrronium bromide), Oxitropium bromide, Pirenzepine, telenzepine or Tiotropium bromide.

In one aspect of the invention the muscarinic receptor antagonist is a long acting muscarinic receptor antagonist, i.e. a muscarinic receptor antagonist with activity that persists for more than 12 hours. Examples of long acting muscarinic receptor antagonists include tiotropium bromide.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is Tiotropium bromide.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a p38 inhibitor, for example, a compound from WO 2005/042502, 681323, 856553, AMG548 (2-[[(2S)-2-amino-3-phenylpropyl]amino]-3-methyl-5-(2-naphthalenyl)-6-(4-pyridinyl)-4(3H)-pyrimidinone), Array-797, Doramapimod, KC-706, PH 797804, R1503, SC-80036, SCIO469 6-chloro-5-[[(2*S*,5*R*)-4-[(4-fluorophenyl)methyl]-2,5-domethyl-1-piperazinyl]carbonyl]-*N*,*N*,1-trimethyl-α-oxo-1*H*-indole-3-acetamide, VX702 or VX745 (5-(2,6-dichlorophenyl)-2-(phenylthio)-6H-pyrimido[1,6-b]pyridazin-6-one).

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a PDE Inhibitor: such as a PDE4 inhibitor {for example, 256066, Arofylline (3-(4-chlorophenyl)-3,7-dihydro-1-propyl-1H-Purine-2,6-dione), AWD 12-281 (N-(3,5-dichloro-4-pyridinyl)-1-[(4-fluorophenyl)methyl]-5-hydroxy-α-oxo-1H-indole-3-acetamide), BAY19-8004 (Bayer), CDC-801 (Calgene), Celgene compound ((βR)-β-(3,4-dimethoxyphenyl)-1,3-dihydro-1-oxo-2H-isoindole-2-propanamide), Cilomilast (cis-4-cyano-4-[3-(cyclopentyloxy)-4-methoxyphenyl]-cyclohexanecarboxylic acid), a compound in WO2006098353 from Kyowa Hakko Kogyo Co. Ltd. Japan, 2-(3,5-dichloro-4-pyridinyl)-1-(7-methoxyspiro[1,3-benzodioxole-2,1'-cyclopentan]-4-yl)ethanone (CAS number 185406-34-2)), Compound from Pfizer (2-(3,4-difluorophenoxy)-5-fluoro-N-[cis-4-[(2-hydroxy-5-methylbenzoyl)amino]cyclohexyl]-)-3-pyridinecarboxamide), Compound from Pfizer (2-(3,4-difluorophenoxy)-5-fluoro-N-[cis-4-[[2-hydroxy-5-(hydroxymethyl)benzoyl]amino]cyclohexyl]-3-pyridinecarboxamide,), CT2820, GPD-1116, Ibudilast, IC 485, KF 31334, KW-4490 (Kyowa Hakko Kogyo), Lirimilast ([2-(2,4-dichlorobenzoyl)-6-[(methylsulfonyl)oxy]-3-benzofuranyl])-urea), Merck Compound (N-cyclopropyl-1,4-dihydro-4-oxo-1-[3-(3-pyridinylethynyl)phenyl]-)-1,8-naphthyridine-3-carboxamide), Oglemilast (N-(3,5-dichloro-4-pyridinyl)-4-(difluoromethoxy)-8-[(methylsulfonyl)amino])-1-dibenzofurancarboxamide), ON06126, ORG 20241 (4-(3,4-dimethoxyphenyl)-N-hydroxy-)-2-thiazolecarboximidamide), PD189659/PD168787 (Parke-Davis), Pentoxifylline (3,7-dihydro-3,7-dimethyl-1-(5-oxohexyl)-)-1H-purine-2,6-dione), Pfizer compound (5-fluoro-N-[4-[(2-hydroxy-4-methylbenzoyl)amino]cyclohexyl]-2-(thian-4-yloxy)pyridine-3-carboxamide), Pfizer UK 500,001, Piclamilast (3-(cyclopentyloxy)-N-(3,5-dichloro-4-pyridinyl)-4-methoxybenzamide), PLX-369 (WO 2006026754), Roflumilast (3-(cyclopropylmethoxy)-N-(3,5-dichloro-4-pyridinyl)-4-(difluoromethoxy)benzamide), SCH 351591 (N-(3,5-dichloro-1-oxido-4-pyridinyl)-8-methoxy-2-(trifluoromethyl)-5-quinolinecarboxamide), SelCID(TM) CC-10004 (Calgene), T-440 (Tanabe), Tetomilast (6-[2-(3,4-diethoxyphenyl)-4-thiazolyl]-2-pyridinecarboxylic acid), Tofimilast (9-cyclopentyl-7-ethyl-6,9-dihydro-3-(2-thienyl)-5H-pyrazolo[3,4-c]-1,2,4-triazolo[4,3-a]pyridine), TPI 1100, UCB 101333-3 (N,2-dicyclopropyl-6-(hexahydro-1H-azepin-1-yl)-5-methyl-4-pyrimidinamine), V-11294A (Napp), VM554/VM565 (Vernalis), or Zardaverine (6-[4-(difluoromethoxy)-3-methoxyphenyl]-3(2H)-pyridazinone); or a PDE5 Inhibitor, for example, Gamma-glutamyl[s-(2-iodobenzyl)cysteinyl]glycine, Tadalafil, Vardenafil, sildenafil, 4-phenyl-methylamino-6-chloro-2-(1-imidazolyl)-quinazoline, 4-phenyl-methylamino-6-chloro-2-(3-pyridyl)-quinazoline, 1,3-dimethyl-6-(2-propoxy-5-methanesulphonylamidophenyl)-1,5-dihydropyrazolo[3,4-d]pyrimidin-4-one or 1-cyclopentyl-3-ethyl-6-(3-ethoxy-4-pyridyl)-pyrazolo[3,4-d]pyrimidin-4-one}.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a PDE4 inhibitor, for example, 256066, Arofylline (3-(4-chlorophenyl)-3,7-dihydro-1-propyl-1H-Purine-2,6-dione), AWD 12-281 (N-(3,5-dichloro-4-pyridinyl)-1-[(4-fluorophenyl)methyl]-5-hydroxy-a-oxo-1H-indole-3-acetamide), BAY19-8004 (Bayer), CDC-801 (Calgene), Celgene compound ((βR)-β-(3,4-dimethoxyphenyl)-1,3-dihydro-1-oxo-2H-isoindole-2-propanamide), Cilomilast (cis-4-cyano-4-[3-(cyclopentyloxy)-4-methoxyphenyl]-cyclohexanecarboxylic acid), a compound in WO2006098353 from Kyowa Hakko Kogyo Co. Ltd. Japan, 2-(3,5-dichloro-4-pyridinyl)-1-(7-methoxyspiro[1,3-benzodioxole-2,1'-cyclopentan]-4-yl)ethanone (CAS number 185406-34-2)), Compound from Pfizer (2-(3,4-difluorophenoxy)-5-fluoro-N-[cis-4-[(2-hydroxy-5-methylbenzoyl)amino]cyclohexyl]-)-3-pyridinecarboxamide), Compound from Pfizer (2-(3,4-difluorophenoxy)-5-fluoro-N-[cis-4-[[2-hydroxy-5-(hydroxymethyl)benzoyl]amino]cyclohexyl]-3-pyridinecarboxamide,), CT2820, GPD-1116, Ibudilast, IC 485, KF 31334, KW-4490 (Kyowa Hakko Kogyo), Lirimilast ([2-(2,4-dichlorobenzoyl)-6-[(methylsulfonyl)oxy]-3-benzofuranyl])-urea), Merck Compound (N-cyclopropyl-1,4-dihydro-4-oxo-1-[3-(3-pyridinylethynyl)phenyl]-)-1,8-naphthyridine-3-carboxamide), Oglemilast (N-(3,5-dichloro-4-pyridinyl)-4-(difluoromethoxy)-8-[(methylsulfonyl)amino])-1-dibenzofurancarboxamide), ON06126, ORG 20241 (4-(3,4-dimethoxyphenyl)-N-hydroxy-)-2-thiazolecarboximidamide), PD189659/PD168787 (Parke-Davis), Pentoxifylline (3,7-dihydro-3,7-dimethyl-1-(5-oxohexyl)-)-1H-purine-2,6-dione), Pfizer compound (5-fluoro-N-[4-[(2-hydroxy-4-methylbenzoyl)amino]cyclohexyl]-2-(thian-4-yloxy)pyridine-3-carboxamide), Pfizer UK 500,001, Piclamilast (3-(cyclopentyloxy)-N-(3,5-dichloro-4-pyridinyl)-4-methoxybenzamide), PLX-369 (WO 2006026754), Roflumilast (3-(cyclopropylmethoxy)-N-(3,5-dichloro-4-pyridinyl)-4-(difluoromethoxy)benzamide), SCH 351591 (N-(3,5-dichloro-1-oxido-4-pyridinyl)-8-methoxy-2-(trifluoromethyl)-5-quinolinecarboxamide), SelCID(TM) CC-10004 (Calgene), T-440 (Tanabe), Tetomilast (6-[2-(3,4-diethoxyphenyl)-4-thiazolyl]-2-pyridinecarboxylic acid), Tofimilast (9-cyclopentyl-7-ethyl-6,9-dihydro-3-(2-thienyl)-5H-pyrazolo[3,4-c]-1,2,4-triazolo[4,3-a]pyridine), TPI 1100, UCB 101333-3 (N,2-dicyclopropyl-6-(hexahydro-1H-azepin-1-yl)-5-methyl-4-pyrimidinamine), V-11294A (Napp), VM554/VM565 (Vernalis), or Zardaverine (6-[4-(difluoromethoxy)-3-methoxyphenyl]-3(2H)-pyridazinone).

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is a PDE4 inhibitor, for example AWD 12-281 (N-(3,5-dichloro-4-pyridinyl)-1-[(4-fluorophenyl)methyl]-5-hydroxy-a-oxo-1H-indole-3-acetamide) or roflumilast.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is roflumilast.

The first active ingredient and the second active ingredient of the pharmaceutical product of the present invention may be administered simultaneously, sequentially or separately to treat respiratory diseases. By simultaneously is meant that the active ingredients are in admixture, or they could be in separate chambers of the same inhaler. By sequential it is meant that the active ingredients are administered, in any order, one immediately after the other. They still have the desired effect if they are administered separately, but when administered in this manner they are generally administered less than 4 hours apart, conveniently less than two hours apart, more conveniently less than 30 minutes apart and most conveniently less than 10 minutes apart, for example less than 10 minutes but not one immediately after the other.

The active ingredients of the present invention may be administered by oral or parenteral (e.g. intravenous, subcutaneous, intramuscular or intraarticular) administration using conventional systemic dosage forms, such as tablets, capsules, pills, powders, aqueous or oily solutions or suspensions, emulsions and sterile injectable aqueous or oily solutions or suspensions. The active ingredients may be delivered to the lung and/or airways via oral administration in the form of a solution, suspension, aerosol or dry powder formulation. These dosage forms will usually include one or more pharmaceutically acceptable ingredients which may be selected, for example, from an adjuvant, carrier, binder, lubricant, diluent, stabilising agent, buffering agent, emulsifying agent, viscosity-regulating agent, surfactant, preservative, flavouring or colorant. As will be understood by those skilled in the art, the most appropriate method of administering the active ingredients is dependent on a number of factors.

In another embodiment the first and second active ingredients are administered via a single pharmaceutical composition (that is, the first and second active ingredients are in admixture). Therefore, the present invention further provides a pharmaceutical composition comprising, in admixture, a first active ingredient which is *N*-[2-(Diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient as defined above. The pharmaceutical composition optionally further comprises a pharmaceutically acceptable adjuvant, diluent or carrier.

The pharmaceutical compositions of the present invention can be prepared by mixing the first active ingredient with the second active ingredient and a pharmaceutically acceptable adjuvant, diluent or carrier. Therefore, in a further aspect of the present invention there is provided a process for the preparation of a pharmaceutical composition, which comprises mixing the first and second active ingredients and a pharmaceutically acceptable adjuvant, diluent or carrier.

It will be understood that the therapeutic dose of each active ingredient administered in accordance with the present invention will vary depending upon the particular active ingredient employed, the mode by which the active ingredient is to be administered, and the condition or disorder to be treated.

In one embodiment of the present invention, the first active ingredient is administered via inhalation. When administered via inhalation the dose of the first active ingredient will generally be in the range of from 0.1 microgram (µg) to 5000 µg, 0.1 to 1000 µg, 0.1 to 500 µg, 0.1 to 100 µg, 0.1 to 50 µg, 0.1 to 5 µg, 5 to 5000 µg, 5 to 1000 µg, 5 to 500 µg, 5 to 100 µg, 5 to 50 µg, 5 to 10 µg, 10 to 5000 µg, 10 to 1000 µg, 10 to 500 µg, 10 to 100 µg, 10 to 50 µg, 20 to 5000 µg, 20 to 1000 µg, 20 to 500 µg, 20 to 100 µg, 20 to 50 µg, 50 to 5000 µg, 50 to 1000 µg, 50 to 500 µg, 50 to 100 µg, 100 to 5000 µg, 100 to 1000 µg or 100 to 500 µg. The dose will generally be administered from 1 to 4 times a day, conveniently once or twice a day, and most conveniently once a day.

In one embodiment of the present invention the second active ingredient is administered by inhalation. When administered via inhalation the dose of the second active ingredient will generally be in the range of from 0.1 microgram (µg) to 5000 µg, 0.1 to 1000 µg, 0.1 to 500 µg, 0.1 to 100 µg, 0.1 to 50 µg, 0.1 to 5 µg, 5 to 5000 µg, 5 to 1000 µg, 5 to 500 µg, 5 to 100 µg, 5 to 50 µg, 5 to 10 µg, 10 to 5000 µg, 10 to 1000 µg, 10 to 500 µg, 10 to 100 µg, 10 to 50 µg, 20 to 5000 µg, 20 to 1000 µg, 20 to 500 µg, 20 to 100 µg, 20 to 50 µg, 50 to 5000 µg, 50 to 1000 µg, 50 to 500 µg, 50 to 100 µg, 100 to 5000 µg, 100 to 1000 µg or 100 to 500 µg. The dose will generally be administered from 1 to 4 times a day, conveniently once or twice a day, and most conveniently once a day.

In another embodiment the present invention provides a pharmaceutical product wherein the molar ratio of first active ingredient to second active ingredient is from 1:1000 to 1000:1, such as from 1:100 to 100:1, for example from 1:50 to 50:1, for example 1:20 to 20:1.

In one embodiment, the present invention provides a pharmaceutical product comprising, in combination, a first active ingredient as defined above, and a second active ingredient as defined above, wherein each active ingredient is formulated for inhaled administration. In a further aspect of this embodiment, the pharmaceutical product is in the form of a pharmaceutical composition comprising the first and second active ingredients in admixture, and which composition is formulated for inhaled administration.

The active ingredients of the present invention are conveniently delivered via oral administration by inhalation to the lung and/or airways in the form of a solution, suspension, aerosol or dry powder (such as an agglomerated or ordered mixture) formulation. For example a metered dose inhaler device may be used to administer the active ingredients, dispersed in a suitable propellant and with or without an additional excipient such as ethanol, a surfactant, lubricant or stabilising agent. A suitable propellant includes a hydrocarbon, chlorofluorocarbon or a hydrofluoroalkane (e.g. heptafluoroalkane) propellant, or a mixture of any such propellants, for example in a pressurised metered dose inhaler (pMDI). Preferred propellants are P134a and P227, each of which may be used alone or in combination with other another propellant and/or surfactant and/or other excipient. A nebulised aqueous suspension or, preferably, solution may also be employed, with or without a suitable pH and/or tonicity adjustment, either as a unit-dose or multi-dose formulation. A suitable device for delivering a dry powder is Turbuhaler®.

The pharmaceutical product of the present invention can, for example, be administered: via an inhaler having the first and second active ingredients in separate chambers of the inhaler such that on administration the active ingredients mix in either the mouthpiece of the inhaler or the mouth of a patient or both (for simultaneous use); or, where the first and second active ingredients are in separate inhalers, via separate inhalers (for separate or sequential use); or the first and second active ingredients are in admixture in an inhaler when the inhaler is supplied to a patient (for simultaneous use).

A dry powder inhaler may be used to administer the active ingredients, alone or in combination with a pharmaceutically acceptable carrier (such as lactose), in the later case either as a finely divided powder or as an ordered mixture. The dry powder inhaler may be single dose or multi-dose and may utilise a dry powder or a powder-containing capsule.

Metered dose inhaler, nebuliser and dry powder inhaler devices are well known and a variety of such devices is available.

The pharmaceutical product of the present invention may be used to treat diseases of the respiratory tract such as obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis, and chronic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus.

Accordingly, the present invention further provides a pharmaceutical product according to the invention for simultaneous, sequential or separate use in therapy.

The present invention further provides the use of a pharmaceutical product according to the invention in the manufacture of a medicament for the treatment of a respiratory disease, in particular chronic obstructive pulmonary disease, asthma, rhinitis, emphysema or bronchitis (such as chronic obstructive pulmonary disease or asthma; for example chronic obstructive pulmonary disease).

The present invention still further provides a method of treating a respiratory disease which comprises simultaneously, sequentially or separately administering:
(a) a therapeutically effective dose of a first active ingredient as defined above; and,
(b) a therapeutically effective dose of a second active ingredient as defined above; to a patient in need thereof.

In a further aspect the present invention provides the use of a pharmaceutical product, kit or composition as hereinbefore described for the treatment of a respiratory disease, in particular chronic obstructive pulmonary disease, asthma, rhinitis, emphysema or bronchitis (such as chronic obstructive pulmonary disease or asthma; for example chronic obstructive pulmonary disease).

In the context of the present specification, the term "therapy" also includes "prophylaxis" unless there are specific indications to the contrary. The terms "therapeutic" and "therapeutically" should be construed accordingly. Prophylaxis is expected to be particularly relevant to the treatment of persons who have suffered a previous episode of, or are otherwise considered to be at increased risk of, the condition or disorder in question. Persons at risk of developing a particular condition or disorder generally include those having a family history of the condition or disorder, or those who have been identified by genetic testing or screening to be particularly susceptible to developing the condition or disorder.

### General Preparative Methods

There follow preparative methods for *N*-[2-(Diethylamino)ethyl]-*N*-(2- {[2-(4-hydroxy-2-oxo-2,3-dihydro-1,3-benzothiazol-7-yl)ethyl]amino}ethyl)-3-[2-(1-naphthyl)ethoxy]propanamide dihydrobromide (Preparations 1 and 2) and also assays and data showing the activity of this compound (called Compound A in the assays and Table 1 below).

¹H NMR spectra were recorded on a Varian *Inova* 400 MHz or a Varian *Mercury*-VX 300 MHz instrument. The central peaks of chloroform-*d* (δ_{H} 7.27 ppm), dimethylsulfoxide-*d₆* (δ_{H} 2.50 ppm), acetonitrile-*d₃* (δ_{H} 1.95 ppm) or methanol-*d₄* (δ_{H} 3.31 ppm) were used as internal references. Column chromatography was carried out using silica gel (0.040-0.063 mm, Merck). Unless stated otherwise, starting materials were commercially available. All solvents and commercial reagents were of laboratory grade and were used as received.

The following method was used for LC/MS analysis:
Instrument Agilent 1100; Column Waters Symmetry 2.1 x 30 mm; Mass APCI; Flow rate 0.7 ml/min; Wavelength 254 nm; Solvent A: water + 0.1% TFA; Solvent B: acetonitrile + 0.1% TFA; Gradient 15-95%/B 8 min, 95% B 1 min.

Analytical chromatography was run on a Symmetry C₁₈-column, 2.1 x 30 mm with 3.5 µm particle size, with acetonitrile/water/0.1% trifluoroacetic acid as mobile phase in a gradient from 5% to 95% acetonitrile over 8 minutes at a flow of 0.7 ml/min.

The abbreviations or terms used in the examples have the following meanings:
- SCX:: Solid phase extraction with a sulfonic acid sorbent
- HPLC:: High performance liquid chromatography
- DMF:: *N*,*N-*Dimethylformamide

### Preparation 1

### N-[2-(Diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide

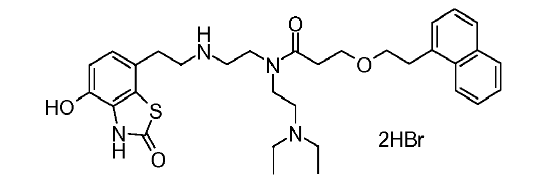

### a) tert-Butyl3-[2-(1-naphthyl)ethoxy]propanoate

1-Naphthalene ethanol (10 g) was treated with benzyltrimethylammonium hydroxide (Triton B^{®}; 0.9 mL of a 40% solution in methanol) and the resulting mixture stirred *in vacuo* for 30 minutes. The mixture was then cooled to 0°C and treated with tert-butyl acrylate (8.19 g). The resulting mixture was slowly warmed to room temperature and stirred overnight. The crude mixture was subsequently absorbed onto aluminium oxide (30 g) and eluted with diethylether (200 mL). The organics were concentrated to give a crude material (16.6 g) which was purified by flash silica chromatography eluting with 1:8, diethylether : hexane to give the subtitled compound (12.83 g).

¹H NMR (CDCl₃) δ 8.05 (dd, 1H), 7.84 (dd, 1H), 7.72 (dd, 1H), 7.54-7.34 (m, 4H), 3.81-3.69 (m, 4H), 3.35 (t, 2H), 2.52-2.47 (m, 2H), 1.45 (s, 9H).

### b) 3-[2-(1-Naphthyl)ethoxy]propanoic acid

*tert*-Butyl 3-[2-(1-naphthyl)ethoxy]propanoate (6.19 g) was taken up in dichloromethane (30 mL) and treated with trifluoroacetic acid (5 mL). The resulting solution was stirred at room temperature for 2 hours, an additional 1 mL of trifluoroacetic acid was added and the solution stirred overnight. The mixture was concentrated, taken up in 2M sodium hydroxide solution (30 mL) and washed with ether (2 x 20 mL). The aqueous layer was subsequently acidified (using 1M hydrochloric acid) and extracted with ether (2 x 30 mL). The combined organics were washed with brine (20 mL), dried over anhydrous magnesium sulphate, filtered and concentrated *in vacuo* to give the sub-titled compound (5.66 g) as a clear oil.

¹H NMR (CDCl₃) δ 8.05 (bs, 1H), 7.85 (bs, 1H), 7.74 (bs, 1H), 7.50-7.38 (m, 4H), 3.84-3.75 (bm, 4H), 3.39 (bs, 2H), 2.65 (bs, 2H).

### c) N-(2-Diethylaminoethyl)-N-(2-hydroxyethyl)-3-[2-(1-naphthyl)ethoxy]-propanamide

Oxalyl chloride (0.33 g) was added dropwise to a solution of 3-[2-(1-naphthyl)ethoxy]propanoic acid (0.53 g) in dichloromethane (10 mL), dimethylformamide (1 drop) was added and stirring continued at room temperature for 1 hour. The mixture was subsequently concentrated, re-dissolved in dichloromethane (10 mL) and added dropwise to a solution of 2-(2-diethylaminoethylamino)ethanol (0.35 g) and diisopropylethylamine (0.56 g) in dichloromethane (10 mL). The resulting mixture was stirred at room temperature for 1 hour, diluted (dichloromethane, 50 mL), washed with water (2 x 20 mL), brine (20 mL), dried over magnesium sulfate and concentrated to give the crude product (0.91 g) which was purified by flash column chromatography (eluting with 5-7% methanol in dichloromethane) to give 0.63 g of the sub-titled compound.

¹H NMR (CDCl₃) δ 8.05 (d, 1H), 7.85 (d, 1H), 7.73 (d, 1H), 7.52-7.47 (m, 2H), 7.42-7.35 (m, 2H), 3.84-3.78 (m, 6H), 3.72-3.70 (m, 1/2H), 3.45-3.35 (m, 6H), 2.79-2.77 (m, 1+1/2H), 2.62-2.58 (m, 2H), 2.54-2.49 (m, 4H), 1.04-1.01 (m, 6H).

### d) N-[2-(Diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide

A solution of dimethylsulfoxide (0.097 g) in dichloromethane (1 mL) was added to a solution of oxalyl chloride (0.079 g) in dichloromethane (10 mL) at -78°C. The reaction was stirred for 15 minutes and then a solution of *N*-(2-diethylaminoethyl)-*N*-(2-hydroxyethyl)-3-[2-(1-naphthyl)ethoxy]propanamide (0.22 g) in dichloromethane (1 mL+ 1mL wash) was added and the reaction mixture stirred for a further 15 minutes. Triethylamine (0.29 g) was added and the reaction allowed to warm to room temperature over 1 hour, the mixture was subsequently diluted (dichloromethane 30 mL), the organics washed with sodium bicarbonate (20 mL), brine (20 mL), dried over anhydrous magnesium sulphate, filtered and concentrated *in vacuo* to give the sub-titled compound (0.21 g).

The crude product was dissolved in methanol (10 mL) and 7-(2-aminoethyl)-4-hydroxy-1,3-benthiazol-2(3H)-one hydrochloride (prepared according to the procedure outlined in Organic Process Research & Development 2004, 8(4), 628-642; 0.131 g) was added along with acetic acid (0.1 mL) and water (0.1 mL). After stirring at room temperature for 30 minutes, sodium cyanoborohydride (0.020 g) was added and the reaction mixture was stirred overnight. Ammonia (7N in methanol, 1 mL) was added and the mixture was concentrated. The crude residue was purified by flash column chromatography eluting with 1% ammonia; 5%-7% methanol in dichloromethane. The crude product was used directly in the next step.

### e) N-[2-(Diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide

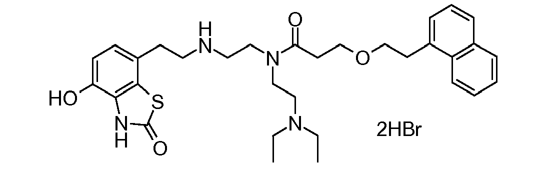

*N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide (0.052 g) was dissolved in ethanol (1.5 mL) and treated with 48 % hydrobromic acid (21 µl). The white solid dihydrobromide salt (0.058 g) was collected by filtration.

MS: APCI(+ve) 579 (M+1)

¹H NMR δ(DMSO) 11.78-11.71 (m, 1H), 10.11-10.06 (m, 1H), 9.51-9.43 (m, 0.33H), 9.21-9.13 (m, 0.66H), 8.75-8.66 (m, 1H), 8.59-8.51 (m, 1H), 8.06 (d, 1H), 7.95-7.90 (m, 1H), 7.79 (d, 1H), 7.60-7.48 (m, 2H), 7.47-7.39 (m, 2H), 6.87 (t, 1H), 6.76 (dd, 1H), 3.78-3.53 (m, 10H), 3.25-3.09 (m, 10H), 2.91-2.80 (m, 2H), 2.73-2.61 (m, 2H), 1.26-1.15 (m, 6H). NMR indicates approximately 2:1 mixture of rotamers at 298K.

### Preparation 2

### N-[2-(Diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide

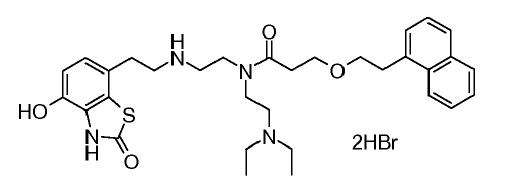

### a) N'-(2,2-Dimethoxyethyl)-N,N-diethyl-ethane-1,2-diamine.

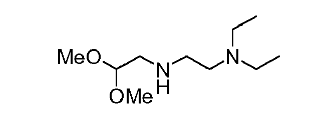

A solution of N,N-diethyl-ethylenediamine (150 g) in methanol (500 mL) was treated dropwise rapidly with glyoxal dimethylacetal (60wt% soln. in water, 225 g) at 10-15°C. After the addition was complete the solution was warmed to 15°C, then to 22°C and left at this temperature for 16 hours. The reaction mixture was treated with 5% palladium on carbon (Johnson-Matthey type 38H paste, 15 g) and hydrogenated at 6 bar until the reaction was complete as judged by GC/MS. The catalyst was removed by filtration and the filtrate evaporated to dryness (toluene azeotrope, 2.5 L), affording 196.2 g of the subtitled compound.

¹H NMR (CDCl₃): 4.48 (t, 1H), 3.39 (s, 6H), 2.75 (d, 2H), 2.69 (t, 2H), 2.57-2.48 (m, 6H), 1.01 (ts, 6H).

### b) N-[2-(Diethylamino)ethyl]-N-(2,2-dimethoxyethyl)-3-[2-(1-naphthyl)ethoxy]propanamide.

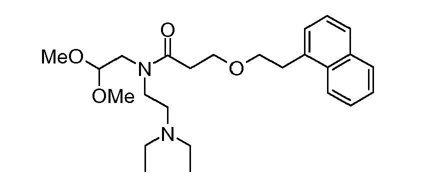

Oxalyl chloride (151 mL) was added dropwise over 45 minutes to a solution of 3-[2-(1-naphthyl)ethoxy]propanoic acid (389 g) (Example 7 step b)) in dichloromethane (2.1 L) and DMF (0.5 mL). The reaction mixture was stirred for a further 16 hours. The mixture was subsequently concentrated, redissolved in DCM (1.7 L) and added dropwise over 1.75 hours at 0°C to a solution of *N*-(2,2-dimethoxyethyl)-*N,N*-diethylethane-1,2-diamine (325 g) and isopropyldiethylamine (551 mL) in DCM (1.7 L). The resulting mixture was stirred at room temperature for 3 hours, washed with aqueous saturated sodium bicarbonate solution (5x1 L), water (1.5 L) and dried over sodium sulphate and concentrated to give 650 g of the sub-titled compound. m/e 431 (M+H⁺, 100%)

### c) N-[2-(Diethylamino)ethyl]-3-[2-(1-naphthyl)ethoxy]-N-(2-oxoethyl)propanamide.

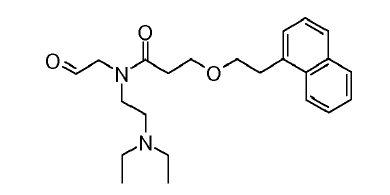

A solution of *N*-[2-(diethylamino)ethyl]-*N*-(2,2-dimethoxyethyl)-3-[2-(1-naphthyl)ethoxy]propanamide (93 g) in DCM (270 mL) was treated dropwise at 0°C with trifluoroacetic acid (270 mL) over 1.5 hours. After the addition the reaction mixture was allowed to warm to room temperature and stirred for a further 1 hour. The reaction mixture was concentrated and the residue poured into aqueous saturated sodium bicarbonate solution (1800 mL, *caution*). The aqueous mixture was extracted with DCM (4x400 mL) and the combined extracts were dried over magnesium sulphate and concentrated. The residue was used directly in the following reaction.

### d) N-[2-(Diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide.

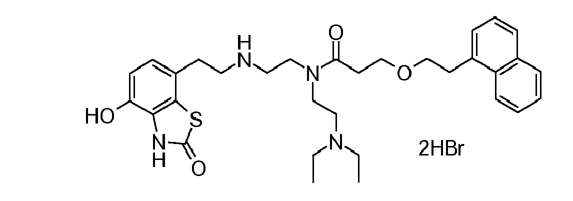

A suspension of 7-(2-amino-ethyl)-4-hydroxy-3H-benzothiazol-2-one hydrochloride (53g) in dry NMP (216 mL) was heated to 60°C and treated in one portion with a solution of NaOH (8.2 g) in methanol (102 mL). The bright orange suspension was cooled to room temperature and treated dropwise with a solution of *N*-[2-(diethylamino)ethyl]-3-[2-(1-naphthyl)ethoxy]-*N*-(2-oxoethyl)propanamide in dichloromethane (475 mL) over 20 minutes. The reaction was left to stir for 25 minutes. Sodium triacetoxyborohydride (91.5 g) was then added in portions over 20 minutes and the mixture stirred for a further 50 minutes. The reaction mixture was poured into water (1.8 L) and the acidic solution (pH5) was washed with tert. butyl methyl ether (TBME) (3x500 mL). The aqueous phase was basified to pH8 by the addition of solid potassium carbonate and extracted with dichloromethane (3x750 mL); the combined organic extracts were dried over magnesium sulphate and concentrated to give a dark oil. This was dissolved in ethanol (200 mL) and 48% aqueous hydrobromic acid (73 mL) was added. The solution was aged for 30 minutes then evaporated to dryness. The residue was triturated with ethanol (560 mL); the resultant solid was collected by filtration and dried in vacuo at 50°C. The sticky solid was suspended in boiling ethanol (100 mL) and filtered while hot. The collected solid was dried in vacuo at 50°C. This material was recrystallised from ethanol/water (3:1, 500 mL). After standing overnight the resultant solid was collected by filtration and washed with ice-cold ethanol (75 mL). Drying in vacuo at 50°C for 24hr afforded 57g of the title compound.

Alternative salts of *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide can be prepared by mixing a suitable acid with *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide in a solvent. Examples are provided below.

### Preparation 3

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide citrate

Citric Acid (248.96 mg) was added to a solution of *N*-[2-(diethylamino)ethyl]-*N*-(2-{[2-(4-hydroxy-2-oxo-2,3-dihydro-1,3-benzothiazol-7-yl)ethyl] amino}ethyl)-3-[2-(1-naphthyl)ethoxy]propanamide (0.5 g) in methanol (5 mL). Immediately, the clear solution became opaque and orange oil settled out. This mixture was heated at an external temp of 60°C forming a clear solution, which was then allowed to cool to room temperature and stirred for 48 h.The resulting precipitate was collected by filtration and washed with methanol (1 mL) and diethyl ether (1 mL). The solid was then dried *in vacuo* at room temperature for 4 h to give the title compound (0.3 g).

¹H NMR (400 MHz, DMSO, 90°C) δ 8.06 (d, 1H), 7.88 (d, 1H), 7.75 (d, 1H), 7.50 (m, 2H), 7.39 (m, 2H), 6.82 (d, 1H), 6.72 (d, 1H), 3.75 (t, 2H), 3.70 (t, 2H), 3.6-3.3 (number of protons could not be determined), 3.28 (t, 2H), 3.1-2.4 (number of protons could not be determined), 0.99 (br, 6H).

### Preparation 4

### N-[2-(dieLhylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide ditosylate

p-Toluenesulfonic acid monohydrate (667.33 mg) was added in one portion to a solution of *N*-[2-(diethylamino)ethyl]-*N*-(2-{[2-(4-hydroxy-2-oxo-2,3-dihydro-1,3-benzothiazol-7-yl)ethyl] amino}ethyl)-3-[2-(1-naphthyl)ethoxy]propanamide (1 g) in methanol (10 mL) producing a clear solution. This was stirred at room temperature for 30 mins then the solvent was removed *in vacuo.* The residue was stirred in diethyl ether (20 mL) at room temperature for 16 h then the solvent was removed and methyl t-butyl ether (20 mL) was added. This mixture was then stirred at room temperature for 16 h before the resulting solid was collected by filtration and washed with methyl t-butyl ether (5 mL). The title compound was dried *in vacuo* at room temperature for 16 h to leave the title compound as a solid (1.18 g).

¹H NMR (400 MHz, DMSO, 90°C) δ 11.35 (1H, br), 8.05 (d, 1H), 7.88 (d, 1H), 7.76 (d, 1H), 7.51 (m, 6H), 7.40 (m, 2H), 7.09 (d, 4H), 6.83 (d, 1H), 6.74 (d, 1H), 3.77 (t, 2H), 3.72 (t, 2H), 3.64 (br, 4H), 3.4-3.0 (number of protons could not be determined), 2.85 (m, 2H), 2.64 (t, 2H), 2.28 (s, 6H), 1.22 (t, 6H).

### Preparation 5

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide phosphate Di-Hemi-Hydrate

Phosphoric Acid (199.19 mg) was added to a solution of *N*-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide (1 g) in methanol (10 mL) producing a gum. The mixture was heated to reflux, and on continued stirring gave a mobile solid. The suspension was allowed to cool slowly to room temperature then filtered and the cake was washed with methanol (2 mL). The title compound (0.93 g) was allowed to dry on the filter.

¹H NMR (400 MHz, DMSO, 90°C) δ 8.05 (d, 1H), 7.87 (d, 1H), 7.74 (m, 1H), 7.48 (m, 2H), 7.38 (m, 2H), 6.78 (d, 1H), 6.68 (d, 1H), 3.73 (t, 2H), 3.67 (t, 2H), 3.31 (m, number of protons could not be determined), 3.26 (t, 2H), 2.8-2.3 (number of protons could not be determined), 0.94 (t, 6H).

### Preparation 6

### N-[2-(diethylamino)ethyl]-N-(2-{[2-(4-hydroxy-2-oxo-2,3-dihydro-1,3-benzothiazol-7-yl)ethyl]amino}ethyl)-3-2-(1-naphthyl)ethoxy]propanamide dixinafoate di-hydrate

A suspension of 1-hydroxy-2-naphthoic acid (328.42 mg) in methanol (3 mL) was added to a solution of *N*-[2-(diethylamino)ethyl]-*N*-(2-{[2-(4-hydroxy-2-oxo-2,3-dihydro-1,3-benzothiazol-7-yl)ethyl] amino}ethyl)-3-[2-(1-naphthyl)ethoxy]propan-amide (0.5 g) in methanol (3 mL) and the resulting mixture was heated to reflux then allowed to cool to room temperature and stirred for 16 h. The title compound was filtered, washed with methanol (1 mL) and dried *in vacuo* at room temperature for 1 h (0.47 g).

¹H NMR (400 MHz, DMSO, 90°C) δ 8.24 (d, 2H), 8.04 (d, 1H), 7.87 (d, 1H), 7.75 (m, 5H), 7.50 (m, 4H), 7.40 (m, 4H), 7.11 (d, 2H), 6.83 (d, 1H), 6.73 (d, 1H), 3.75 (t, 2H), 3.70 (t, 2H), 3.6-3.3 (m, 4H), 3.26 (t, 2H), 2.6-3.1 (number of protons could not be determined), 2.59 (t, 2H), 1.05 (br, 6H).

### Preparation 7

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide dixinafoate Di-Hemi-Hydrate

20mg of Di-Hydrate Polymorph A (Preparation 6) was slurried in water (0.5ml) for one week. The resulting suspension was centrifuged and the supernatant was seperated from the solid material, the latter being left to air dry overnight in a fume hood.

### Preparation 8

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide sulfate

Concentrated sulphuric acid (23.98 µL) was added dropwise to a solution of *N*-[2-(diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide (0.25 g) in methanol (2.5 mL). The mixture was stirred at room temperature for 20 mins then heated at an external temperature of 60°C then allowed to cool back to room temperature. Methyl t-butyl ether (0.5 mL) was then added and the mixture was heated at an external temperature of 60°C then allowed to room temperature. The mixture was transferred to another flask using methanol to dissolve the mixture and then the solvent was removed *in vacuo.* Methyl t-butyl ether (10 mL) was added to the residue and the mixture was stirred at room temperature for 16 h. The title compound was collected by filtration and dried on the filter (0.24 g).

¹H NMR (400 MHz, DMSO, 90°C) δ 8.05 (m, 1H), 7.88 (m, 1H), 7.75 (m, 1H), 7.50 (m, 2H), 7.38 (m, 2H), 6.85 (m, 1H), 6.73 (m, 1H), 3.74 (m, 4H), 3.58 - 3.40 (m, number of protons could not be determined), 3.28 (t, 2H), 3.03 - 2.73 (m, number of protons could not be determined), 2.60 (t, 2H), 1.09 (s, 6H).

### Preparation 9

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide mono benzoate

Benzoic acid (52.75 mg) was added in one portion to a solution of *N*-[2-(diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide (0.25 g) in methanol (2.5 mL) producing a clear solution. This was stirred at room temperature for 1 h then the solvent was removed *in vacuo.* The residue was stirred in acetonitrile (5 mL) at room temperature for 16 h then the solvent was removed and methyl t-butyl ether (10 mL) was added. This mixture was stirred at room temperature for 3 h before the title compound was collected by filtration. The title compound was isolated as a solid.

¹H NMR (400 MHz, DMSO, 90°C) δ 8.04 (m, 1H), 7.94 (m, 2H), 7.87 (m, 1H), 7.73 (m, 1H), 7.57 - 7.34 (m, 7H), 6.84 - 6.68 (m, 2H), 3.80 - 3.54 (m, 6H), 3.30 (m, could not be established due to overlap with water), 2.78 - 2.37 (m, number of protons could not be determined), 0.91 (m, 6H).

### Preparation 10

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide mono benzoate as a crystalline form

20mg of the solid mono-benzoate salt from Preparation 9 was dissolved in 1ml of propan-2-ol. The resulting solution was left to evaporate slowly at room temperature in a fume hood, leaving an off-white solid.

### Preparation 11

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide mono fumarate

Fumaric acid (168.31 mg) was added to a solution of *N*-[2-(diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide (0.84 g) in methanol (2 mL) producing an opaque mixture. The mixture was warmed at an external temperature of 60°C then allowed to cool to room temperature and stirred for 16 h. The title compound was obtained as a foam after evaporation to dryness.

¹H NMR (400 MHz, DMSO, 90°C) δ 8.05 (m, 1H), 7.87 (m, 1H), 7.74 (m, 1H), 7.49 (m, 2H), 7.38 (m, 2H), 6.80 (m, 1H), 6.70 (m, 1H), 6.58 (s, 2H), 3.78 - 3.53 (m, 6H), 3.36 - 3.19 (m, number of protons could not be determined), 2.82 - 2.40 (m, number of protons could not be determined), 0.96 - 0.86 (m, 6H).

### Preparation 12

### N-[2-(diethylamino)ethyl]-N-(2-{[2-(4-hydroxy-2-oxo-2,3-dihydro-1,3-benzothiazol-7-yl)ethyl]amino}ethyl)-3-[2-(1-naphthyl)ethoxy]propanamide mono besylate

Benzenesulfonic acid (158.51 mg) was added to a solution of *N*-[2-(diethylamino)ethyl]-*N-*(2- {[2-(4-hydroxy-2-oxo-2,3-dihydro-1,3-benzothiazol-7-yl)ethyl]amino}ethyl)-3-[2-(1-naphthyl)ethoxy]propanamide (0.58 g) in methanol (5.8 mL) producing an clear solution. The mixture was stirred at room temperature for 1 h. The title compound was obtained as a solid after evaporation to dryness.

¹H NMR (400 MHz, DMSO, 90°C) δ 8.05 (d, 1H), 7.88 (d, 1H), 7.75 (m, 1H), 7.64 (m, 2H), 7.50 (m, 2H), 7.40 (m, 2H), 7.28 (m, 3H), 6.83 (m, 1H), 6.73 (m, 1H), 3.77 - 3.35 (m, number of protons could not be determined), 3.28 (t, 2H), 3.01 - 2.47 (m, number of protons could not be determined), 1.05 (br, 6H).

### Adrenergic β2 mediated cAMP production

### Cell preparation

H292 cells were grown in 225cm2 flasks incubator at 37°C, 5% CO₂ in RPMI medium containing, 10% (v/v) FBS (foetal bovine serum) and 2 mM L-glutamine.

### Experimental Method

Adherent H292 cells were removed from tissue culture flasks by treatment with Accutase^{™} cell detachment solution for 15 minutes. Flasks were incubated for 15 minutes in a humidified incubator at 37°C, 5% CO₂. Detached cells were re-suspended in RPMI media (containing 10% (v/v) FBS and 2 mM L-glutamine) at 0.05 x 10⁶ cells per mL. 5000 cells in 100 µL were added to each well of a tissue-culture-treated 96-well plate and the cells incubated overnight in a humidified incubator at 37°C, 5% CO₂. The culture media was removed and cells were washed twice with 100 µL assay buffer and replaced with 50 µL assay buffer (HBSS solution containing 10mM HEPES pH7.4 and 5 mM glucose). Cells were rested at room temperature for 20 minutes after which time 25 µL of rolipram (1.2 mM made up in assay buffer containing 2.4% (v/v) dimethylsulphoxide) was added. Cells were incubated with rolipram for 10 minutes after which time Compound A was added and the cells were incubated for 60 minutes at room temperature. The final rolipram concentration in the assay was 300 µM and final vehicle concentration was 1.6% (v/v) dimethylsulphoxide. The reaction was stopped by removing supernatants, washing once with 100 µL assay buffer and replacing with 50 µL lysis buffer. The cell monolayer was frozen at -80°C for 30 minutes (or overnight).

### AlphaScreen^{™} cAMP detection

The concentration of cAMP (cyclic adenosine monophosphate) in the cell lysate was determined using AlphaScreen^{™} methodology. The frozen cell plate was thawed for 20 minutes on a plate shaker then 10 µL of the cell lysate was transferred to a 96-well white plate. 40 µL of mixed AlphaScreen^{™} detection beads pre-incubated with biotinylated cAMP, was added to each well and the plate incubated at room temperature for 10 hours in the dark. The AlphaScreen^{™} signal was measured using an EnVision spectrophotometer (Perkin-Elmer Inc.) with the recommended manufacturer's settings. cAMP concentrations were determined by reference to a calibration curve determined in the same experiment using standard cAMP concentrations. A concentration response curve for Compound A was constructed and data was fitted to a four parameter logistic equation to determine both the pEC₅₀ and Intrinsic Activity. Intrinsic Activity was expressed as a fraction relative to the maximum activity determined for formoterol in each experiment. A result for Compound A is in Table 1.

### Selectivity Assays

### Adrenergic α1D

### Membrane Preparation

Membranes were prepared from human embryonic kidney 293 (HEK293) cells expressing recombinant human α1D receptor. These were diluted in Assay Buffer (50mM HEPES, 1mM EDTA, 0.1% gelatin, pH 7.4) to provide a final concentration of membranes that gave a clear window between maximum and minimum specific binding.

### Experimental Method

Assays were performed in U-bottomed 96-well polypropylene plates. 10 µL [³H]-prazosin (0.3 nM final concentration) and 10 µL of Compound A (10x final concentration) were added to each test well. For each assay plate 8 replicates were obtained for [³H]-prazosin binding in the presence of 10 µL vehicle (10% (v/v) DMSO in Assay Buffer; defining maximum binding) or 10µL BMY7378 (10 µM final concentration; defining non-specific binding (NSB)). Membranes were then added to achieve a final volume of 100 µL. The plates were incubated for 2 hours at room temperature and then filtered onto PEI coated GF/B filter plates, pre-soaked for 1 hour in Assay Buffer, using a 96-well plate Tomtec cell harvester. Five washes with 250 µL wash buffer (50mM HEPES, 1mM EDTA, pH 7.4) were performed at 4°C to remove unbound radioactivity. The plates were dried then sealed from underneath using Packard plate sealers and MicroScint-O (50 µL) was added to each well. The plates were sealed (TopSeal A) and filter-bound radioactivity was measured with a scintillation counter (TopCount, Packard BioScience) using a 3-minute counting protocol.

Total specific binding (B₀) was determined by subtracting the mean NSB from the mean maximum binding. NSB values were also subtracted from values from all other wells. These data were expressed as percent of B₀. Compound concentration-effect curves (inhibition of [³H]-prazosin binding) were determined using serial dilutions typically in the range 0.1 nM to 10 µM. Data was fitted to a four parameter logistic equation to determine the compound potency, which was expressed as pIC50 (negative log molar concentration inducing 50% inhibition of [³H]-prazosin binding). Result is shown in Table 1 below.

### Adrenergic β1

### Membrane Preparation

Membranes containing recombinant human adrenergic beta 1 receptors were obtained from Euroscreen. These were diluted in Assay Buffer (50mM HEPES, 1mM EDTA, 120mM NaCl, 0.1 % gelatin, pH 7.4) to provide a final concentration of membranes that gave a clear window between maximum and minimum specific binding.

### Experimental Method

Assays were performed in U-bottomed 96-well polypropylene plates. 10 µL [¹²⁵I]-Iodocyanopindolol (0.036 nM final concentration) and 10 µL of Compound A (10x final concentration) were added to each test well. For each assay plate 8 replicates were obtained for [¹²⁵I]-Iodocyanopindolol binding in the presence of 10 µL vehicle (10% (v/v) DMSO in Assay Buffer; defining maximum binding) or 10 µL Propranolol (10 µM final concentration; defining non-specific binding (NSB)). Membranes were then added to achieve a final volume of 100 µL. The plates were incubated for 2 hours at room temperature and then filtered onto PEI coated GF/B filter plates, pre-soaked for 1 hour in Assay Buffer, using a 96-well plate Tomtec cell harvester. Five washes with 250 µL wash buffer (50mM HEPES, 1mM EDTA, 120mM NaCl, pH 7.4) were performed at 4°C to remove unbound radioactivity. The plates were dried then sealed from underneath using Packard plate sealers and MicroScint-O (50 µL) was added to each well. The plates were sealed (TopSeal A) and filter-bound radioactivity was measured with a scintillation counter (TopCount, Packard BioScience) using a 3-minute counting protocol.

Total specific binding (B₀) was determined by subtracting the mean NSB from the mean maximum binding. NSB values were also subtracted from values from all other wells. These data were expressed as percent of B₀. Compound concentration-effect curves (inhibition of [¹²⁵I]-Iodocyanopindolol binding) were determined using serial dilutions typically in the range 0.1 nM to 10 µM. Data was fitted to a four parameter logistic equation to determine the compound potency, which was expressed as pIC₅ₒ (negative log molar concentration inducing 50% inhibition of [¹²⁵I]-Iodocyanopindolol binding). A result is shown in Table 1 below.

### Dopamine D2

### Membrane Preparation

Membranes containing recombinant human Dopamine Subtype D2s receptors were obtained from Perkin Elmer. These were diluted in Assay Buffer (50mM HEPES, 1mM EDTA, 120mM NaCl, 0.1 % gelatin, pH 7.4) to provide a final concentration of membranes that gave a clear window between maximum and minimum specific binding.

### Experimental Method

Assays were performed in U-bottomed 96-well polypropylene plates. 30 µL [³H]-spiperone (0.16 nM final concentration) and 30 µL of Compound A (10x final concentration) were added to each test well. For each assay plate 8 replicates were obtained for [³H]-spiperone binding in the presence of 30 µL vehicle (10% (v/v) DMSO in Assay Buffer; defining maximum binding) or 30 µL Haloperidol (10 µM final concentration; defining non-specific binding (NSB)). Membranes were then added to achieve a final volume of 300 µL. The plates were incubated for 2 hours at room temperature and then filtered onto PEI coated GF/B filter plates, pre-soaked for 1 hour in Assay Buffer, using a 96-well plate Tomtec cell harvester. Five washes with 250 µL wash buffer (50mM HEPES, 1mM EDTA, 120mM NaCl, pH 7.4) were performed at 4°C to remove unbound radioactivity. The plates were dried then sealed from underneath using Packard plate sealers and MicroScint-O (50 µL) was added to each well. The plates were sealed (TopSeal A) and filter-bound radioactivity was measured with a scintillation counter (TopCount, Packard BioScience) using a 3-minute counting protocol.

Total specific binding (B₀) was determined by subtracting the mean NSB from the mean maximum binding. NSB values were also subtracted from values from all other wells. These data were expressed as percent of B₀. Compound concentration-effect curves (inhibition of [³H]-spiperone binding) were determined using serial dilutions typically in the range 0.1 nM to 10 µM. Data was fitted to a four parameter logistic equation to determine the compound potency, which was expressed as pIC₅₀ (negative log molar concentration inducing 50% inhibition of [³H]-spiperone binding). A result for Compound A is shown in Table 1.

**Table 1**

| Compound | β2 pEC50 | β2 Int Act | α1 bind pIC50 | β1 bind p IC50 | D2 bind pIC50 |
|---|---|---|---|---|---|
| A | 8.2 | 0.8 | 6.6 | <5 | 6.1 |

The present invention will now be further explained by reference to the following illustrative Example. Example 1 refers to the following Figure:

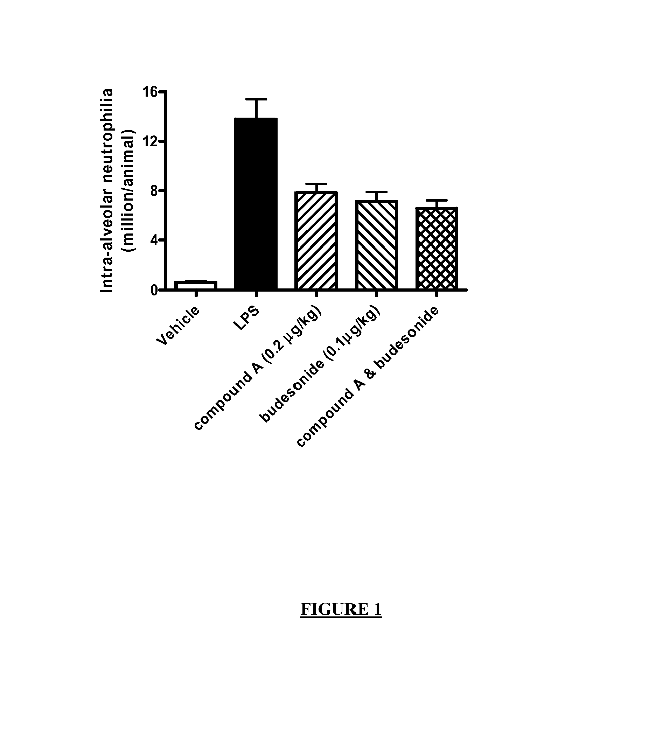

Figure 1. LPS- induced intra-alveolar neutrophilia in CRL:CD rats after LPS challenge (10µg/kg). Rats were dosed with vehicle, compound A (0.2µg/kg), budesonide (0.1 µg/kg) or a combination of compound A (0.2µg/kg) and budesonide (0.1 µg/kg) 30 min prior to LPS challenge.

### Example 1

### Evaluation of compound activity on intra-alveolar neutrophil migration after intratracheal challenge with lipopolysaccharride (LPS) in the CRL:CD rat.

LPS challenge in CRL:CD rats causes an influx of neutrophils into the lungs. Under recoverable gaseous anaesthesia (5% isoflourane in oxygen), rats were dosed *via* the intatracheal route with vehicle (0.05M phosphate, 0.1 % Tween 80, 0.6% saline, pH 6), or compound 30 min before challenge with an intratracheal dose of 10µg/kg LPS.

The rats (250-400g) were euthanized 4hr after LPS challenge with 1mL pentobarbitone sodium. A tracheotomy was performed and a cannula inserted. The airway was then lavaged using 3 mL Isoton at room temperature. The Isoton (BeckmanCoulter, High Wycombe, UK) was left in the airway for 10 seconds before being removed. The bronchio-alveolar lavage (BAL) fluid containing inflammatory cells was placed into a 15 mL centrifuge tube and kept on ice. This process was repeated three times. An aliquot of BAL fluid was removed and the inflammatory cells were counted on Sysmex (Sysmex UK, Milton Keynes). Neutrophils were expressed in millions/animal (mean ± s.e.mean).

### Compound A and budesonide combination:

Rats were dosed with vehicle, Compound A (0.2µg/kg), budesonide (0.1 µg/kg) or a combination of Compound A (0.2µg/kg) and budesonide (0.1µg/kg). The level of neutrophils in the compound vehicle/saline group was 0.61±0.11 million/animal (n=8) and 14±1.6 million/ animal (n=20) in the compound vehicle/LPS challenge group. Intratracheal administration of compound A at 0.2µg/kg 30 minutes prior to LPS challenge produced 45% inhibition of neutrophilia (7.8±0.71 million/animal, n=20). Budesonide (0.1µg/kg) produced 51% inhibition (7.1±0.77 million/animal, n=19) and the combination of Compound A (0.2µg/kg) and budesonide (0.1µg/kg) produced 55% inhibition of LPS-induced neutrophilia (6.6±0.63 million/animal, n=19) (Figure 1).

## Claims

1. A pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl] amino}ethyl)-3-[2-(1-naphthyl)ethoxy]propanamide or a salt thereof, and a second active ingredient which is a corticosteroid.

2. A kit comprising:
a preparation of a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a salt thereof,
a preparation of a second active ingredient which is a corticosteroid, and optionally,
instructions for the simultaneous, sequential or separate administration of the preparations to a patient in need thereof.

3. A pharmaceutical composition comprising, in admixture:
a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a salt thereof;
a second active ingredient which is a corticosteroid; and,
a pharmaceutically acceptable adjuvant, diluent or carrier.

4. A pharmaceutical product, kit or composition as claimed in claim 1, 2 or 3 wherein the first active ingredient is in the form of a salt which is a hydrochloride, hydrobromide, trifluoroacetate, sulphate, phosphate, acetate, fumarate, maleate, tartrate, lactate, citrate, pyruvate, succinate, oxalate, methanesulphonate, *p-*toluenesulphonate, bisulphate, benzenesulphonate, ethanesulphonate, malonate, xinafoate, ascorbate, oleate, nicotinate, saccharinate, adipate, formate, glycolate, L-lactate, D-lactate, aspartate, malate, L-tartrate, D-tartrate, stearate, 2-furoate, 3-furoate, napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), isethionate (2-hydroxyethylsulfonate), 2-mesitylenesulphonate or 2-naphthalenesulphonate.

5. A pharmaceutical product, kit or composition as claimed in any one of the preceding claims wherein the first active ingredient is in the form of a salt which is a dihydrobromide.

6. A pharmaceutical product, kit or composition as claimed in any one of the preceding claims wherein the second active ingredient is Alclometasone dipropionate, Amelometasone, Beclomethasone dipropionate, Budesonide, Butixocort propionate, Ciclesonide, Clobetasol propionate, Desisobutyrylciclesonide, Etiprednol dicloacetate, Fluocinolone acetonide, Fluticasone Furoate, Fluticasone propionate, Loteprednol etabonate (topical) or Mometasone furoate.

7. A pharmaceutical product, kit or composition as claimed in any one of the preceding claims wherein the second active ingredient is budesonide, fluticasone propionate, fluticasone fruoate, mometasone furoate, beclomethasone dipropionate or butixocort propionate ester.

8. A pharmaceutical product, kit or composition as claimed in any one of the preceding claims wherein the second active ingredient is budesonide.

9. A pharmaceutical product as claimed in any one of claims 1 to 5 comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl] amino}ethyl)-3-[2-(1-naphthyl)ethoxy]propanamide dihydrobromide, and a second active ingredient is Budesonide, Fluticasone Furoate or Fluticasone propionate.

10. A pharmaceutical product, kit or composition as claimed in any one of claims 1 to 9 for use in therapy.

11. Use of a pharmaceutical product, kit or composition as claimed in any one of claims 1 to 9 in the manufacture of a medicament for the treatment of a respiratory disease.

12. A pharmaceutical product, kit or composition as claimed in any one of claims 1 to 9 for use in the treatment of a respiratory disease.

13. Use according to claim 11 or 12, wherein the respiratory disease is chronic obstructive pulmonary disease, asthma, rhinitis, emphysema or bronchitis.

14. Use according to claim 11 or 12, wherein the respiratory disease is chronic obstructive pulmonary disease or asthma.
